# EUROPEAN PATENT SPECIFICATION

(11) **EP 3 463 402 B1**
(45) Date of publication and mention of the grant of the patent: **23.06.2021**
(21) Application number: 17737885.8
(22) Date of filing: 22.05.2017
(51) Int. Cl.: A61K 35/42, A61P 11/00

(54) **METHODS OF USING PULMONARY CELLS FOR TRANSPLANTATION AND INDUCTION OF TOLERANCE**
VERFAHREN ZUR VERWENDUNG VON LUNGENZELLEN ZUR TRANSPLANTATION UND INDUKTION VON TOLERANZ
MÉTHODES D'UTILISATION DE CELLULES PULMONAIRES POUR TRANSPLANTATION ET INDUCTION DE TOLÉRANCE

(30) Priority: 22.05.2016 US 201662339887 P; 23.06.2016 US 201662353709 P
(43) Date of publication of application: 10.04.2019
(73) Proprietor: Yeda Research and Development Co. Ltd., 7610002 Rehovot (IL)
(72) Inventor: REISNER, Yair, 6803775 Old Jaffa (IL); HILLEL-KARNIEL, Carmit, 7610002 Rehovot (IL); ROSEN, Chava, 7610002 Rehovot (IL); BACHAR-LUSTIG, Esther, 7610002 Rehovot (IL); SHEZEN, Elias, 7610002 Rehovot (IL)
(74) Representative: Dennemeyer & Associates S.A.
(86) International application number: PCT/IL2017/050569
(87) International publication number: WO 2017/203520

(56) References cited:
- WO-A1-2013/084190
- CHEN J ET AL: "Enrichment of hematopoietic stem cells with SLAM and LSK markers for the detection of hematopoietic stem cell function in normal and Trp53 null mice", EXPERIMENTAL HEMATOLOGY, ELSEVIER INC, US, vol. 36, no. 10, 1 October 2008 (2008-10-01), pages 1236-1243, XP025465827, ISSN: 0301-472X, DOI: 10.1016/J.EXPHEM.2008.04.012 [retrieved on 2008-06-17]
- MEGAN SYKES: "Hematopoietic Cell Transplantation for Tolerance Induction: Animal Models to Clinical Trials :", TRANSPLANTATION, vol. 87, no. 3, 1 February 2009 (2009-02-01), pages 309-316, XP055396593, GB ISSN: 0041-1337, DOI: 10.1097/TP.0b013e31819535c2

## Description

### FIELD AND BACKGROUND OF THE INVENTION

The present invention, in some embodiments thereof, relates to lung tissue cells in suspension comprising hematopoietic progenitor cells for use in therapeutic applications.

Respiratory diseases are the second leading cause of death in the word. Most currently available therapies only slightly improve the quality of life of lung disease patients, and do not prevent the loss of gas-exchange surface, which is a major consequence of progression in a variety of pulmonary pathologies. Thus, the best way to cure end-stage lung disease is by organ transplantation. However, shortage of organs results in the death of many patients while on the waiting list.

During the past decade, the potential curative role of stem cell based therapies has been extensively investigated. Recent findings suggest that early progenitors derived from adult tissues, such as the bone marrow or from the umbilical cord blood, amniotic fluid or placenta, including mesenchymal stem cells, endothelial progenitors or circulating fibrocytes and a variety of other populations, could structurally engraft and differentiate as airways and alveolar epithelial cells or as vascular endothelial or interstitial lung cells and could be utilized in repair and regeneration of injured or diseased lungs [Baber SR et al., American Journal of Physiology-Heart and Circulatory Physiology. (2007) 292(2): H1120; Weiss DJ. Pulm Pharmacol Ther. (2008) 21(4):588-94; Weiss DJ et al., Proceedings of the American thoracic society: Am Thoracic Soc; (2008) p. 637; Sueblinvong V and Weiss DJ. Translational Research. (2010) 156(3): 188-205]. However, lack of significant epithelial transdifferentiation, the extremely complex structure of the lung, comprised of more than 40 different cell types, and a low engraftment rate of transplanted cells in the lung, in different experimental models, represent a major challenge.

Recently, the group of Reisner Y. showed that canalicular embryonic mouse and human lung tissues are enriched with several lung progenitors [Rosen, C. et al., Nat Med (2015) 21(8): p. 869-79]. According to these studies, upon adequate pre-conditioning, creating a space in the host lung stem cell niche, intravenous infusion of a single cell suspension of canalicular lung cells induced marked long-term lung chimerism which can heal injured lungs.

Additional background art includes PCT Publication No. WO 2013/084190 and PCT Publication No. WO/2013/093920.

### SUMMARY OF THE INVENTION

According to an aspect of some embodiments of the present invention there is provided non-syngeneic pulmonary tissue cells in suspension comprising an effective amount of hematopoietic precursor cells (HPCs) or supplemented with HPCs, wherein the effective amount is a sufficient amount to achieve tolerance to the pulmonary tissue cells in the absence of chronic immunosuppressive regimen, and wherein said effective amount of said HPCs comprises at least about 1 x 10⁵ per Kg body weight of a subject, for use in treating a pulmonary disorder or injury in a subject in need thereof or for inducing donor specific tolerance in a subject in need of a pulmonary cell or tissue transplantation.

According to some embodiments of the invention, the non-syngeneic pulmonary tissue cells in suspension for use further comprises a sublethal, lethal or supralethal conditioning protocol.

According to some embodiments of the invention, the conditioning protocol comprises reduced intensity conditioning (RIC).

According to some embodiments of the invention, the conditioning protocol comprises *in-vivo* T cell debulking.

According to some embodiments of the invention, the *in-vivo* T cell debulking is effected by antibodies.

According to some embodiments of the invention, the antibodies comprise an anti-CD8 antibody, an anti-CD4 antibody, or both.

According to some embodiments of the invention, the antibodies comprise anti-thymocyte globulin (ATG) antibodies, anti-CD52 antibodies or anti-CD3 (OKT3) antibodies.

According to some embodiments of the invention, the conditioning protocol comprises at least one of total body irradiation (TBI), total lymphoid irradiation (TLI), partial body irradiation, a chemotherapeutic agent and/or an antibody immunotherapy.

According to some embodiments of the invention, the TBI comprises a single or fractionated irradiation dose within the range of 1-10 Gy.

According to some embodiments of the invention, the non-syngeneic pulmonary tissue cells in suspension for use further comprises an agent capable of inducing damage to the pulmonary tissue, wherein the damage results in proliferation of resident stem cells in the pulmonary tissue.

According to some embodiments of the invention, the agent capable of inducing damage to the pulmonary tissue is selected from the group consisting of a chemotherapeutic agent, an immunosuppressive agent, an amiodarone, a beta blockers, an ACE inhibitor, a nitrofurantoin, a procainamide, a quinidine, a tocainide, and a minoxidil.

According to some embodiments of the invention, the agent capable of inducing damage to the pulmonary tissue comprises naphthalene.

According to some embodiments of the invention, the non-syngeneic pulmonary tissue cells in suspension for use further comprises the use of an immunosuppressive agent for up to two weeks following the non-syngeneic pulmonary tissue cells in suspension.

According to some embodiments of the invention, the method further comprises treating the subject with an immunosuppressive agent for up to two weeks following the administering.

According to some embodiments of the invention, the immunosuppressive agent comprises cyclophosphamide.

According to some embodiments of the invention, the cyclophosphamide is for a single dose administration.

According to some embodiments of the invention, the cyclophosphamide is for a two dose administration.

According to some embodiments of the invention, the cyclophosphamide is administered in a single dose.

According to some embodiments of the invention, the cyclophosphamide is administered in two doses.

According to some embodiments of the invention, each of the two doses comprises a concentration of about 50-150 mg per kg body weight.

According to some embodiments of the invention, each of the two doses is effected on days 3 and 4 following the non-syngeneic pulmonary tissue cells in suspension.

According to some embodiments of the invention, each of the two doses is administered on days 3 and 4 following the administering.

According to some embodiments of the invention, the pulmonary tissue cells comprise mammalian pulmonary cells.

According to some embodiments of the invention, the mammalian pulmonary cells are human cells.

According to some embodiments of the invention, the pulmonary tissue cells comprise fetal pulmonary cells.

According to some embodiments of the invention, the fetal pulmonary cells are from a fetus at a developmental stage corresponding to that of a human pulmonary organ/tissue at a gestational stage selected from a range of about 20 to about 22 weeks of gestation.

According to some embodiments of the invention, the pulmonary tissue cells comprise adult pulmonary cells.

According to some embodiments of the invention, the adult pulmonary cells are obtained from a cadaver.

According to some embodiments of the invention, the adult pulmonary cells are obtained from a living donor.

According to some embodiments of the invention, the pulmonary tissue cells comprise de-differentiated cells.

According to some embodiments of the invention, the pulmonary tissue cells comprise *ex vivo* expanded cells.

According to some embodiments of the invention, the non-syngeneic pulmonary tissue is allogeneic with respect to the subject.

According to some embodiments of the invention, the non-syngeneic pulmonary tissue is xenogeneic with respect to the subject.

According to some embodiments of the invention, the pulmonary tissue cells in suspension are at a dose of at least about 10 x 10⁶ cells per kilogram body weight of the subject.

According to some embodiments of the invention, the pulmonary tissue cells in suspension are at a dose of at least about 40 x 10⁶ cells per kilogram body weight of the subject.

According to some embodiments of the invention, the pulmonary tissue cells in suspension are at a dose of at least about 100 x 10⁶ cells per kilogram body weight of the subject.

According to some embodiments of the invention, the pulmonary tissue cells in suspension are administered at a dose of at least about 10 x 10⁶ cells per kilogram body weight of the subject.

According to some embodiments of the invention, the pulmonary tissue cells in suspension are administered at a dose of at least about 40 x 10⁶ cells per kilogram body weight of the subject.

According to some embodiments of the invention, the pulmonary tissue cells in suspension are administered at a dose of at least about 100 x 10⁶ cells per kilogram body weight of the subject.

According to some embodiments of the invention, the pulmonary tissue cells in suspension are depleted of T cells.

According to some embodiments of the invention, the HPCs comprise Lin⁻ Sca-1⁺ c-kit⁺ (LSK) cells and/or signaling lymphocytic activation molecule (SLAM) cells.

According to some embodiments of the invention, the HPCs comprise at least about 1 x 10⁶ cells per kilogram body weight of the subject.

According to some embodiments of the invention, the HPCs comprise at least about 10 x 10⁶ cells per kilogram body weight of the subject.

According to some embodiments of the invention, the supplemented with the HPCs comprises the addition of HPCs obtained from the same donor as the pulmonary tissue.

According to some embodiments of the invention, the supplemented with the HPCs comprises the addition of HPCs obtained from a different donor than the pulmonary tissue.

According to some embodiments of the invention, the supplemented with the HPCs comprises the pulmonary tissue cells in the same formulation as the HPCs.

According to some embodiments of the invention, the supplemented with the HPCs comprises the pulmonary tissue cells in a separate formulation than the HPCs.

According to some embodiments of the invention, the supplemented with the HPCs comprises administering the pulmonary tissue cells in the same formulation as the HPCs.

According to some embodiments of the invention, the supplemented with the HPCs comprises administering the pulmonary tissue cells in a separate formulation than the HPCs.

According to some embodiments of the invention, the pulmonary tissue cells in suspension comprise a heterogeneous population of cells.

According to some embodiments of the invention, the heterogeneous population of cells comprises any of hematopoietic progenitor cells, epithelial progenitor cells, mesenchymal progenitor cells and/or endothelial progenitor cells.

According to some embodiments of the invention, the non-syngeneic pulmonary tissue cells in suspension for use are formulated for an intravenous route or an intratracheal route of administration.

According to some embodiments of the invention, administering is effected by an intravenous route or an intratracheal route.

According to some embodiments of the invention, the subject is a human subject.

According to some embodiments of the invention, the subject in need of the pulmonary cell or tissue transplantation has a pulmonary disorder or injury.

According to some embodiments of the invention, the pulmonary disorder or injury comprises chronic inflammation of the lungs.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

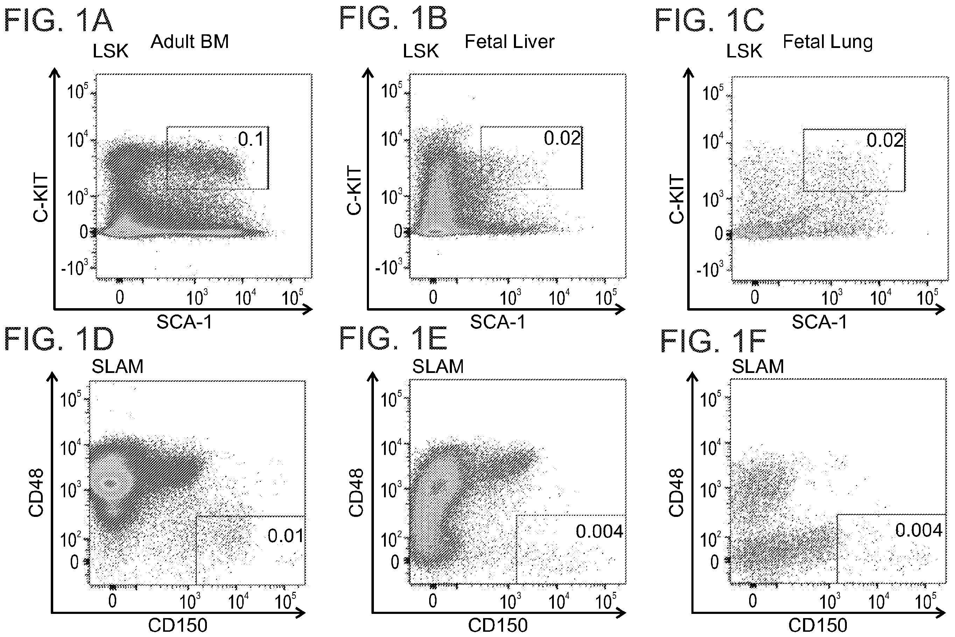

FIGs. 1A-F are graphs illustrating hematopoietic population of LSK and SLAM cells in adult lung, fetal liver and fetal lung. FACS characterization gated on live, single cells, CD45⁺, KSL (lineage⁻, SCA-1⁺, C-KIT⁺) (Figures 1A-C) or SLAM (lineage⁻, CD48⁻, CD150⁺) (Figures 1D-F) hematopoietic stem cells in adult bone marrow (BM) (Figures 1A, 1D), embryonic (E16) liver (Figures 1B, 1E); and embryonic (E16) lung (Figures 1C, 1F).

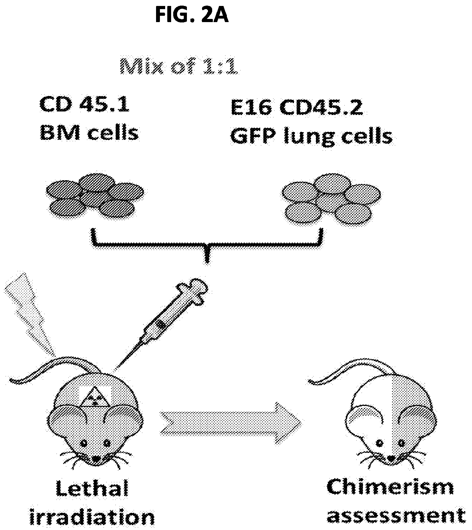

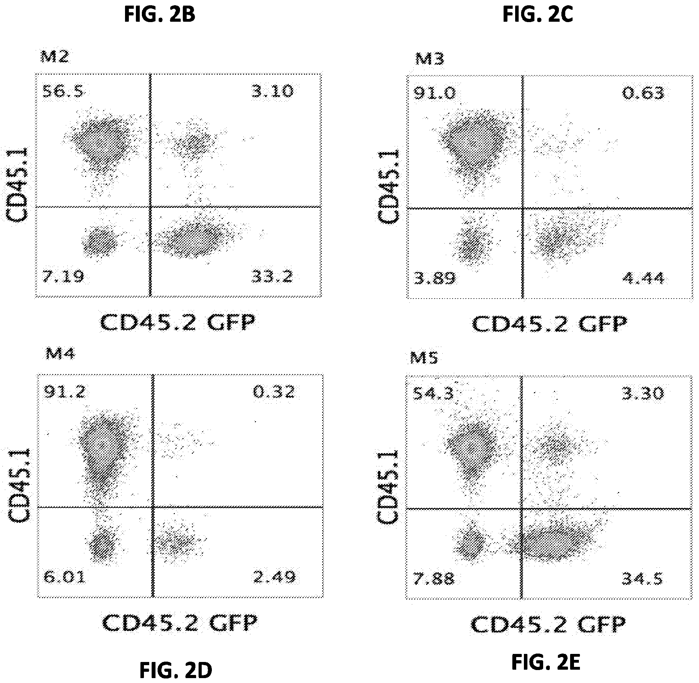

FIGs. 2A-E illustrate that E16 embryonic mouse lung tissue exhibits marked levels of long-term repopulating hematopoietic stem cells. (Figure 2A) The conditioning and transplantation scheme; (Figures 2B-E) FACS analysis showing hematopoietic chimerism in peripheral blood of NA+10 Gy treated animals (n=5) transplanted with E16 lung cells. Mice were infused with a cell mixture of CD45.1 bone marrow and GFP⁺ CD45.2⁺ E16 lung cells at a 1:1 ratio. Chimerism was determined 9 months following transplantation in four different mice.

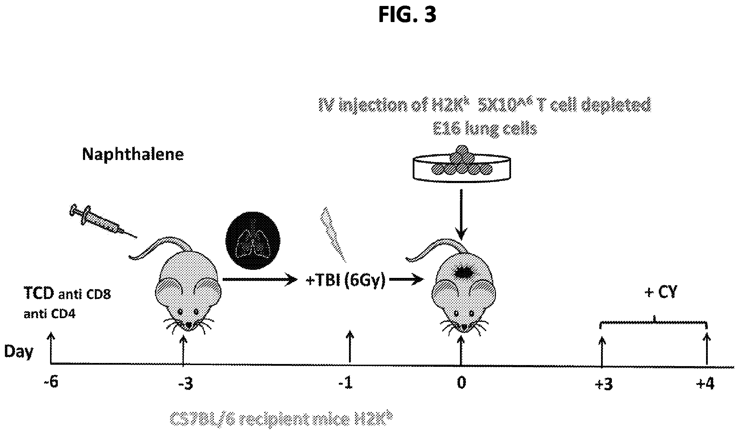

FIG. 3 is a schematic illustration of the conditioning protocol used for allogeneic embryonic lung transplantation. Pre conditioning of C57BL H-2K^{b} recipients: T cell debulking (TCD) with anti-CD4 and anti-CD8 antibodies at day -6 (300 µg each), naphthalene (NA) at day -3, 6 Gy total body irradiation (TBI) at day -1, cyclophosphamide (CY) 100 mg/kg/day at days 3,4 post allogeneic transplantation of donor 'mega dose' (5 x 10⁶) T cell depleted of E15-16 C3H H-2K^{k} lung cells.

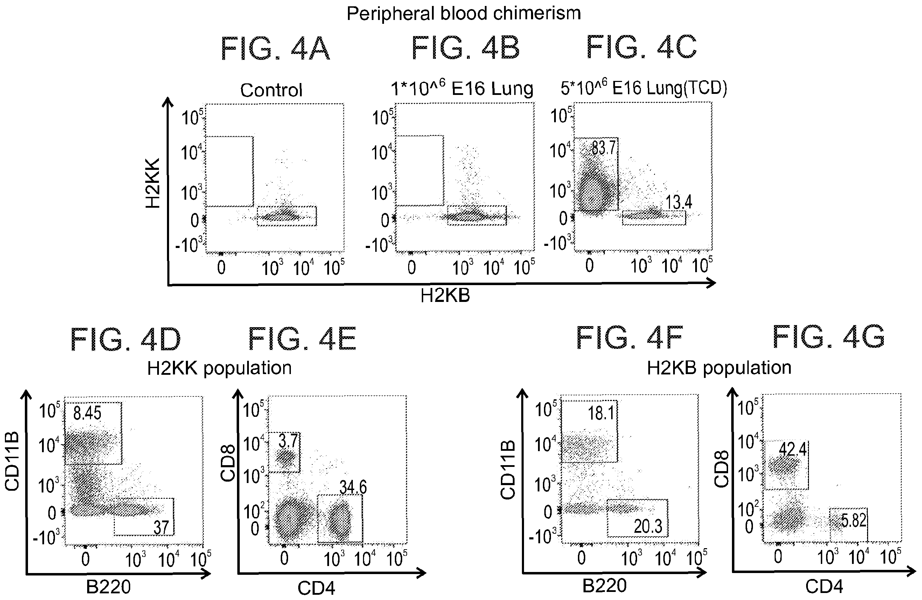

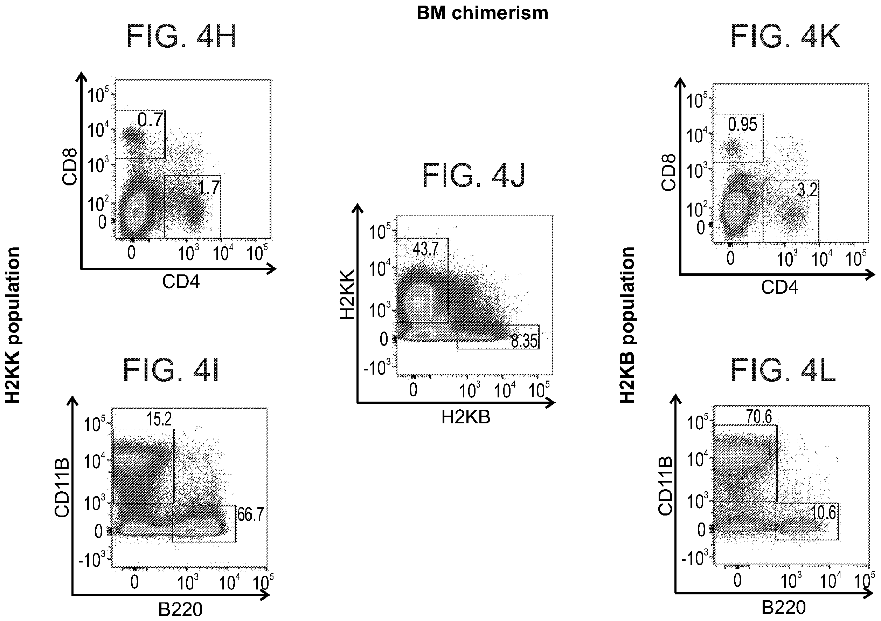

FIGs. 4A-L are graphs illustrating peripheral blood, BM and lung chimerism in the allogeneic 'megadose' T cell depleted lung transplantation. FACS characterization of peripheral blood donor type (C3H H-2K^{k} cells) and recipient type (C57BL H-2K^{b}) chimerism in control mice (no transplantation), regular dose (1 x 10⁶) and 'mega dose' (5 x 10⁶) transplantation of T cell depleted of E15-16 C3H H-2K^{k} lung cells. The recipient mice were conditioned as described in Figure 3. Donor derived H-2K^{k} or host derived H-2K^{b} B220 B cells, CD4/8 T cells, CD11b myeloid cells in peripheral blood and bone marrow are shown in (Figures 4A-G) and (Figures 4H-L), respectively.

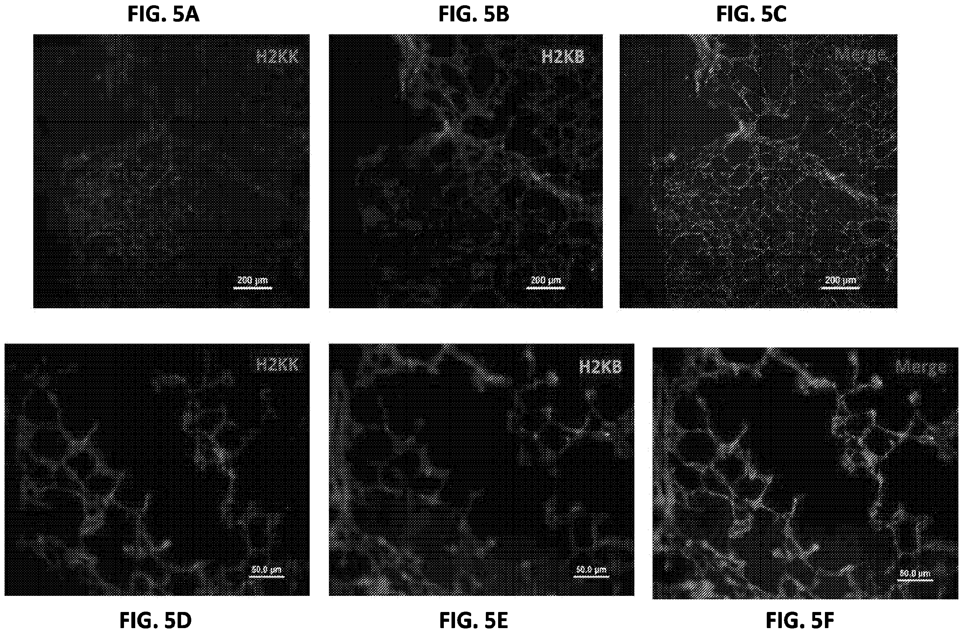

FIGs. 5A-F are photographs illustrating lung chimerism in the allogeneic 'megadose' T cell depleted lung transplantation. The photographs illustrate representative lung immunohistology following establishment of hematopoietic chimerism (as illustrated in Figures 4A-L) depicting donor derived C3H H-2K^{k}-PE red lung "patches" as opposed to host allogeneic C57BL/6 H-2K^{b} cells (green).

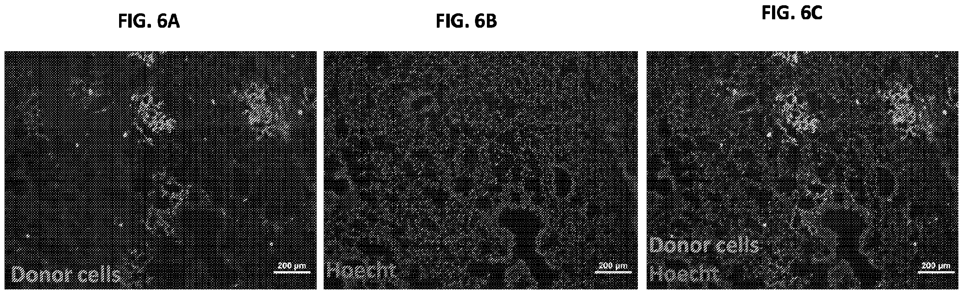

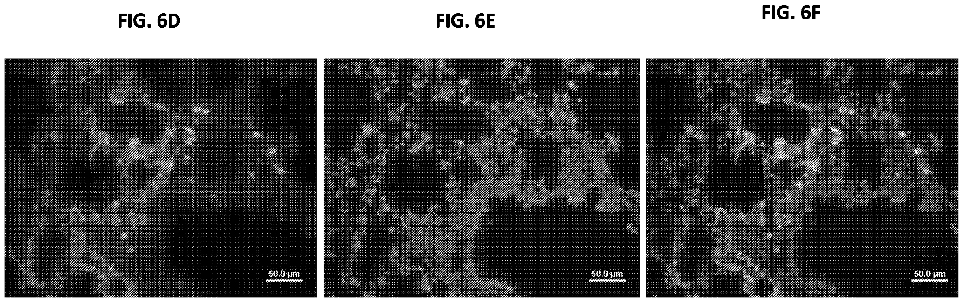

FIGs. 6A-F are photographs depicting lung chimerism induction following transplantation of fresh adult lung cells. A single cell suspension comprising 8 x 10⁶ adult lung cells was harvested from a GFP positive C57BL/6 mouse and injected i.v. into C57BL/6 recipient mice following conditioning with NA and 6 GY TBI. After 8 weeks, lung tissue from transplanted mice was fixed in 4 % PFA and GFP positive patches were determined by immune-histology. Green - donor derived cells; Blue - hoechst staining for nuclei. Figures 6A-C and Figures 6D-F show different magnifications of the same field, respectively.

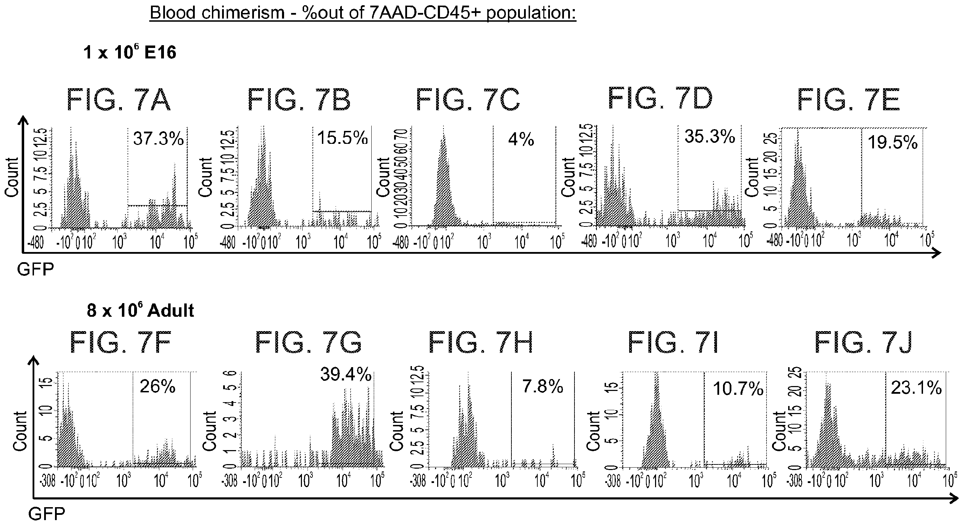

FIGs. 7A-J are graphs depicting induction of hematopoietic chimerism by fresh adult GFP-lung cells 8 weeks after transplantation. Figures 7A-E illustrate chimerism in five mice transplanted with 1 x 10⁶ fetal lung cells E16. Figures 7F-J illustrate chimerism in five mice transplanted with 8 x 10⁶ adult lung cells. Blood chimerism - % out of 7AAD-CD45⁺ population.

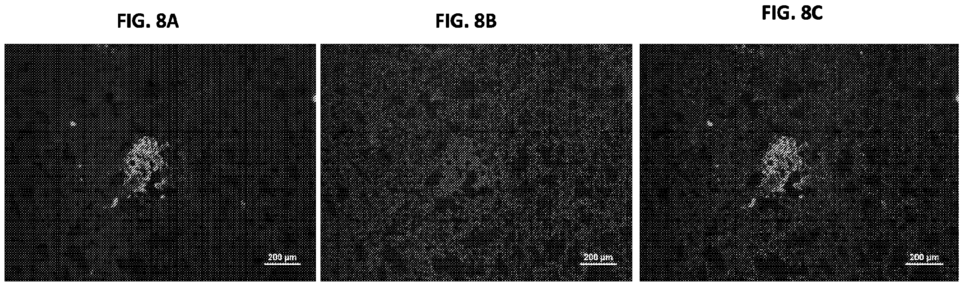

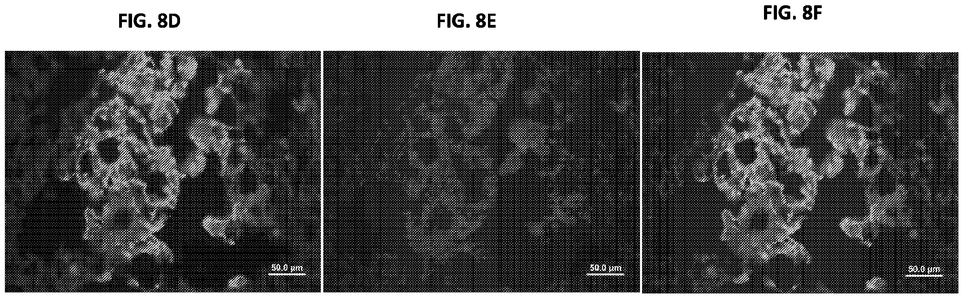

FIGs. 8A-F are photographs depicting lung chimerism induction following transplantation of E16 lung expanded cells: GFP positive C57BL E16 lung cells were harvested and seeded on tissue culture plates with condition medium (irradiated mouse embryonic feeders (iMEF)) together with epithelial growth factor and Rock inhibitor. Medium was changed every 2 days and Rock inhibitor was added freshly every time. After 4 days, cells were passed by splitting them into 3 plates. After 3 additional days of culture, the cells were used for transplantation. A single cell suspension comprising 2 x 10⁶ expanded cells were injected i.v. into C57BL/6 recipient mice following conditioning with NA and 6 GY TBI. After 8 weeks, lung tissue from transplanted mice was fixed in 4 % PFA and GFP positive patches were determined by immune-histology. Green - donor derived cells; Blue - hoechst staining for nuclei. Figures 8A-C and Figures 8D-F show different magnifications of the same field.

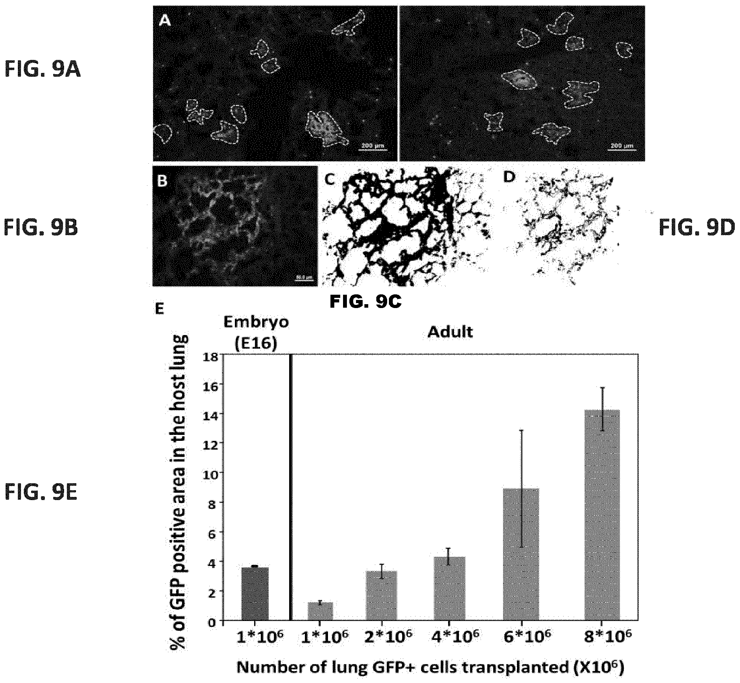

FIGs. 9A-E illustrate morphometric analysis of lung occupancy by donor-derived cells following transplantation of different doses of lung cells. Figure 9A) GFP⁺ patches (circled) in two representative histological lung sections of recipient mice, 8 weeks after transplantation of 4 x 10⁶ GFP⁺ adult lung cells following conditioning. GFP^{low} staining reflects auto-fluorescence of the host lung cells. Figure 9B) A typical GFP⁺ patch further analyzed by Fiji software to calculate the total area of GFP^{high} (donor-derived cells) plus GFP^{low} (autofluorescence of host cells) (Figure 9C), in comparison to GFP^{high} only (Figure 9D). Figure 9E) Percentage of the lung area occupied by GFP^{high} tissue out of the total area occupied by both host GFP^{low} and donor GFP^{high} cells. This script was used automatically on all histology pictures collected from at least seven slides, each containing three cryo-cuts with gap of 36 micron from each other, yielding more than 1,000 micron depth into the lung tissue. Results following transplantation of different numbers of adult lung cells (Right) compared to lung occupancy after transplantation of E16 fetal lung cells (Left) (n=4 mice per each group, 45 to 60 fields per mice).

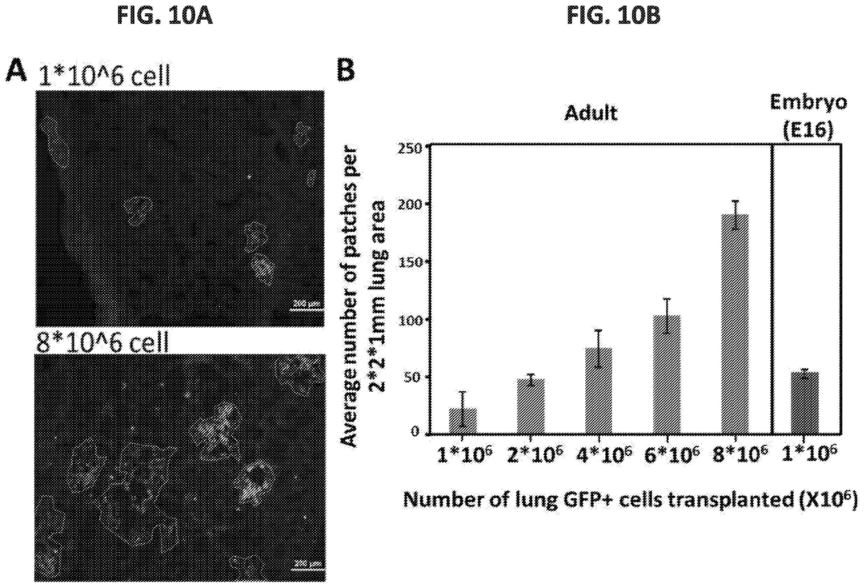

FIGs. 10A-B illustrate patch formation following transplantation of different lung cell doses. Figure 10A) Typical histological sections showing GFP⁺ patches (circled) after transplantation of 1 x 10⁶ or 8 x 10⁶ adult lung cells following host conditioning. Figure 10B) Average number of GFP⁺ patches in 2 x 2 x 1 mm lung area of recipient mice (n=4 mice per each group).

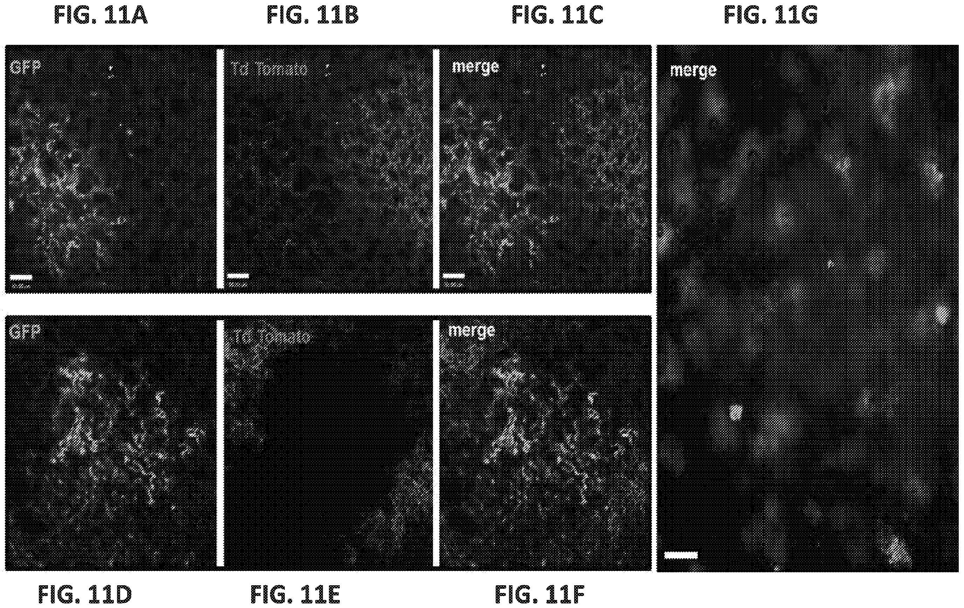

FIGs. 11A-G are photographs of three dimensional analysis by two photon microscopy revealing discrete green or red lung patches after transplantation of a 1:1 mixture of GFP and TdTomato lung cells. 1:1 mixture of GFP and TdTomato adult lung cells was transplanted into recipient mice following conditioning. Mice were sacrificed and examined by 2 photon microscopy 8 weeks after transfer. Discrete green or red patches were found in the host lung. The absence of yellow patches strongly indicates the clonal origin of each patch. Figures 11A-F) illustrate two typical fields at high magnification (Extended focus image showing entire scan depth of chimeric lung; each z step=1µm merge of 30 to 80 planes. Scale bar 50 µm). Figure 11G) image of a larger field at low magnification (Scale bar 200 µm).

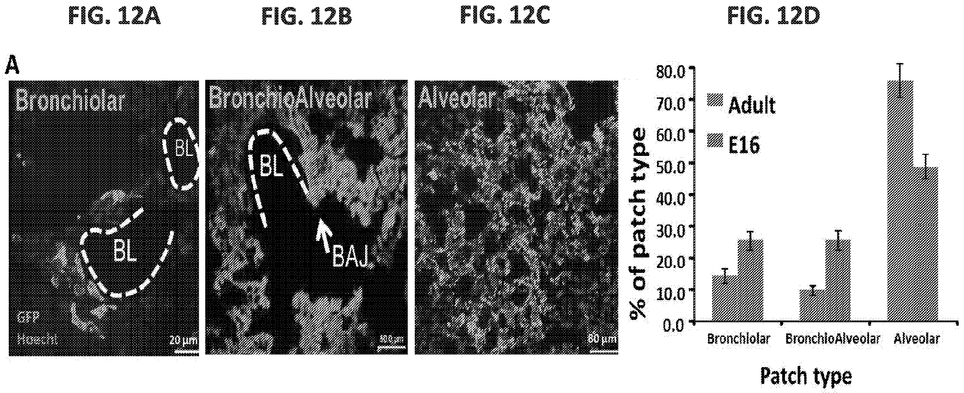

FIGs. 12A-D illustrate different patch types found in the host lung after transplantation. Host mice were sacrificed 8 weeks post-transplantation and their lungs were evaluated by immuno-histology. Figures 12A-C) three major types of patches were identified according to their anatomical position, namely, alveolar, bronchiolar and bronchoalveolar. Bronchiolar lumen (BL) and broncho-alveolar junctions (BAJ) areas are bolded in the figures. Of note, it is possible to see that the GFP positive, donor derived cells, are present in both alveolar and bronchiolar regions in the host lung. Figure 12D) Percentage of patch types out of the total number of patches. These results (n=6 mice, 8 fields were counted for each mouse) were compared to results found after transplantation of 1 x 10⁶ E16 fetal lung cells (n=3 mice, 10 fields were counted for each mouse).

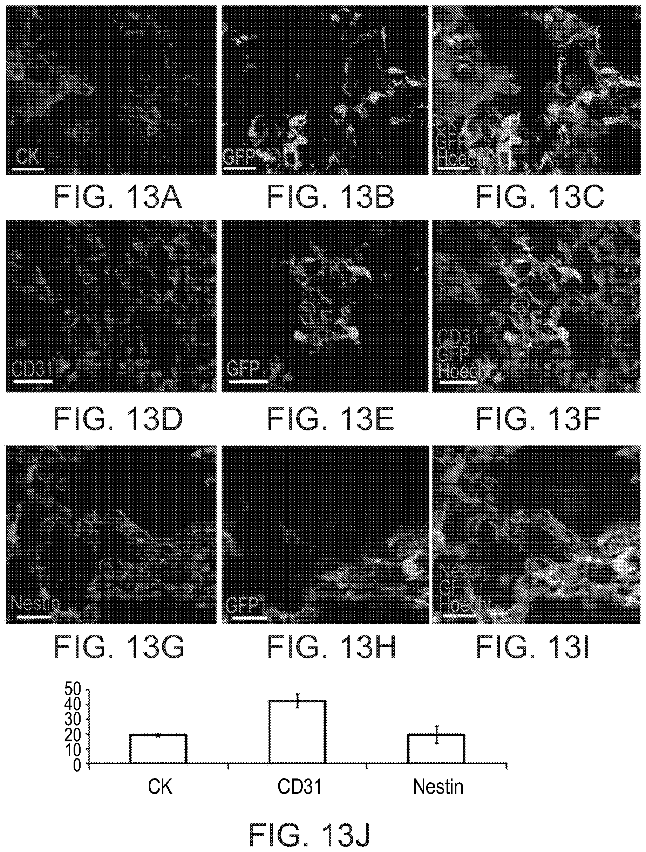

FIGs. 13A-J illustrate different cell lineages found in GFP⁺ donor-derived patches. Immunohistological analysis was used to characterize the composition of donor derived GFP⁺ green patches. Imaris software was used to determine fluorescence co-localization. GFP⁺ areas were set as the area of interest, and the co-localization channel identified pixels that included both red (antibody staining) and green (donor derived cell labeling) fluorescence. Epithelial cells were stained with wide spectrum cytokeratin antibody (Figures 13A-C); endothelial cells were stained with CD31 antibody (Figures 13D-F); and mesenchymal cells were stained with Nestin (Figures 13G-I). In each figure, the Red channel represents the lineage marker, the Green channel marks donor derived GFP⁺ cells, and the Blue marks nuclear staining. Scale bar = 20 µm. Figure 13J) Summary of % ROI (average plus SD) colocalized 8 weeks after transplantation of 8 x 10⁶ adult GFP⁺ lung cells (based on five different areas obtained from two transplanted mice).

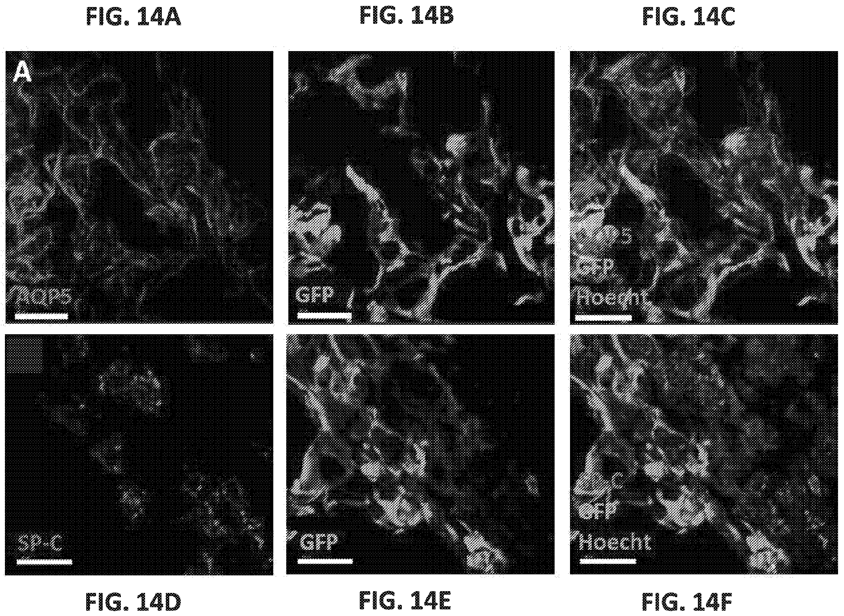

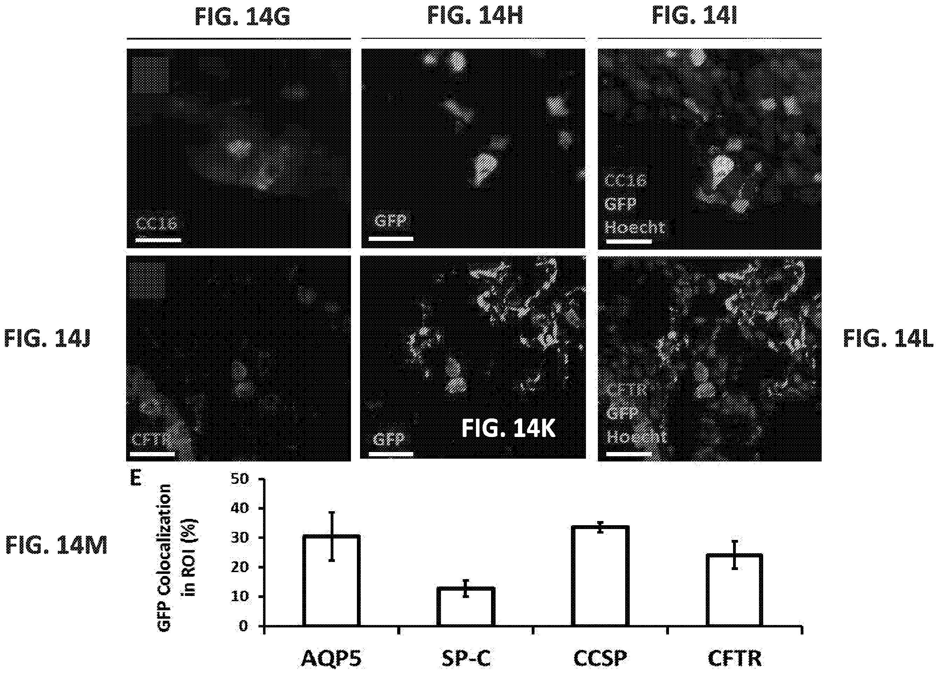

FIGs. 14A-M illustrate integration of donor-derived cells in the epithelial lung compartment. Immunohistological analysis was used to characterize the composition of the epithelial compartment within donor-derived GFP⁺ green patches. Imaris software was used to determine fluorescence co-localization. GFP⁺ areas were designated as the areas of interest, and the co-localization channel identified pixels that included both red and green colors. Alveolar type I cells (ATI) cells were stained with Aquaporin type 5 (Figures 14A-C), alveolar type II (ATII) cells with surfactant protein C (Figures 14D-F), Club cells with CCSP (CC16) (Figures 14G-I), and CFTR+ cells with anti-CFTR (Figures 14J-L). In each figure, the Red channel represents the lineage marker, the Green channel marks donor-derived GFP⁺ cells, and the Blue channel marks nuclear staining. Scale bar = 40 µm. Figure 14M) Summary of % ROI (average plus SD) colocalized 8 weeks after transplantation of 8 x 10⁶ adult GFP⁺ lung cells (based on five different areas obtained from two transplanted mice).

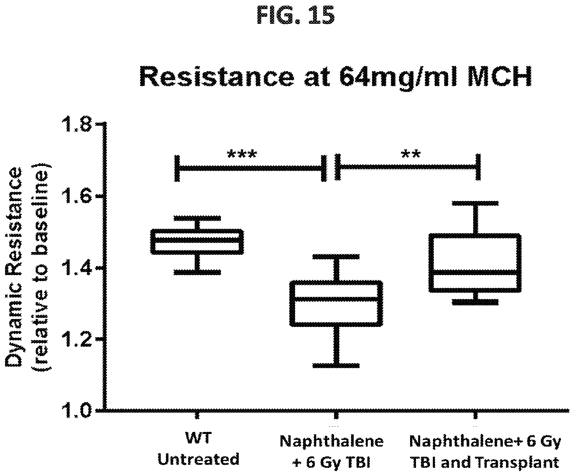

FIG. 15 is a graph illustrating dynamic lung resistance before and after adult lung cell transplantation. Dynamic lung resistance was measured following methacholine challenge (64 mg/ml) using the Scireq-FlexiVent instrument (Emka, France) in wild type untreated C57BL/6 mice (left column), in mice treated with naphthalene and 6 Gy TBI (middle column), and in mice treated with naphthalene and 6 GY TBI and transplanted with 4 x 10⁶ adult lung cells (right column). Significant differences between the three groups were established by the Anova test (n=10 in each group). Box plots show entire data distribution. Center line, median; box limits, 25^{th} and 75^{th} percentiles; whiskers, minimal and maximal values.

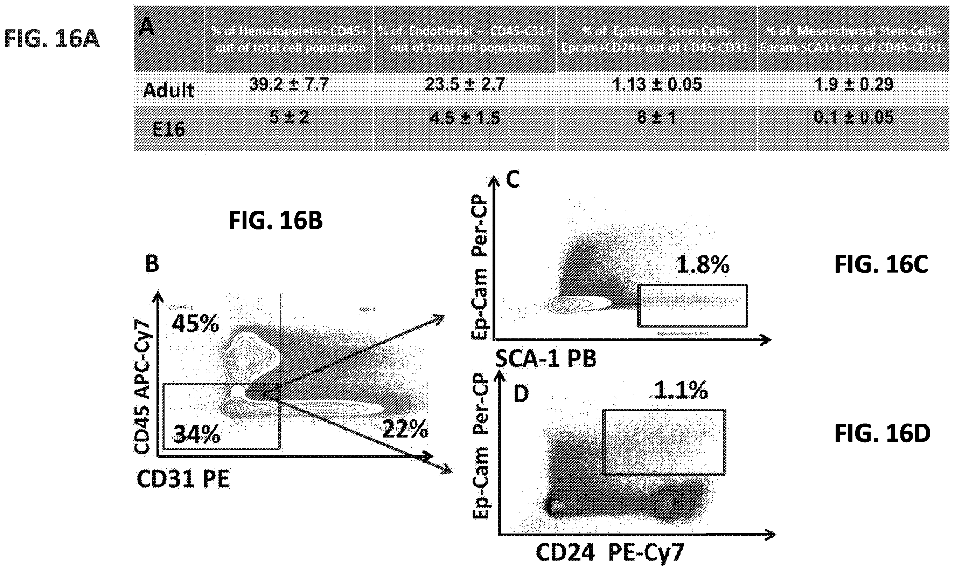

FIGs. 16A-D illustrate FACS analysis of lung progenitors in the adult lung. Fresh adult lung cells were harvested and stained for FACS analysis. Figure 16A) Average percentage of cells from each group out of the total cell population were summarized (n=3) and compared to results from embryo lung from day 16. Figure 16B) Hematopoietic and endothelial populations were stained, and only CD45⁻ and CD31⁻ cells were also stained with the Ep-Cam, epithelial marker in order to distinguish between epithelial stem cells (Ep-Cam⁺CD24⁺) (Figure 16C), and mesenchymal stem cells (Ep-Cam⁻SCA-1⁺) (Figure 16D).

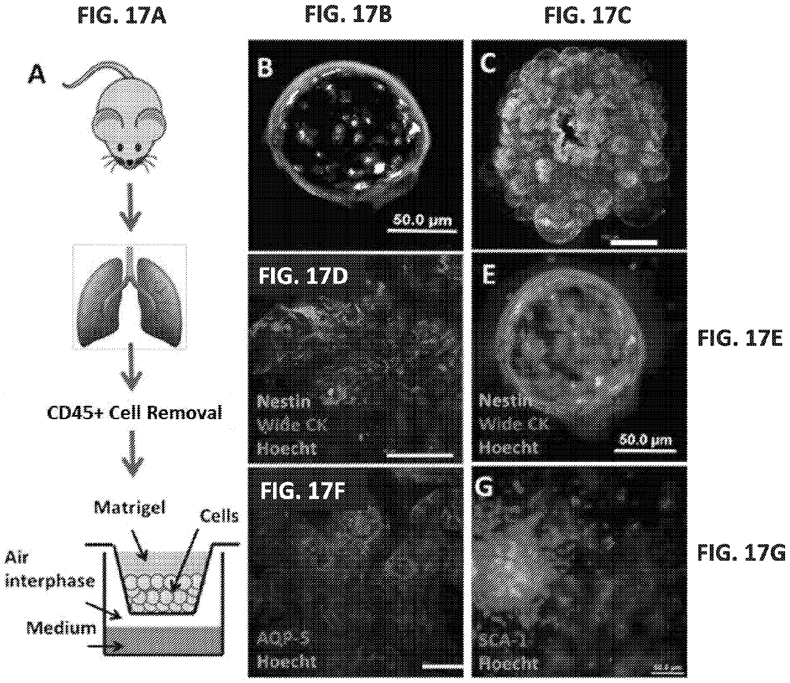

FIGs. 17A-G illustrate *in vitro* 3D differentiation of adult lung progenitors. Figure 17A) Schematic representation of 3D culture. Figures 17B-C) Light phase image of lung organoids after 3 weeks in culture. Two major types of organoids can be seen- bronchiolar like, round structures surrounded by a thick mesenchymal layer of cells (Figure 17B), or alveolar-like, consisting of mostly epithelial cells with round balloon-like shapes (Figure 17C). Organoids express not only epithelial markers, such as wide spectrum cytokeratins (Figures 17D-E) and an alveolar type I cell marker (AQP5) (Figure 17F), but also exhibit a mesenchymal marker (Nestin) (Figures 17D-E), inside the organoid shape and around it (SCA-1) (Figure 17G), probably playing a role as supporting cells.

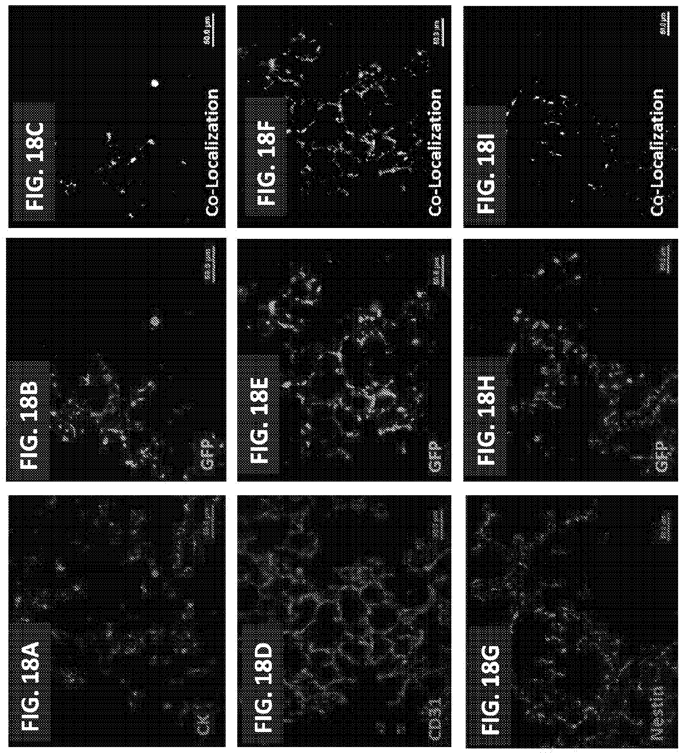

FIGs. 18A-I illustrate different cell lineages found in GFP⁺ donor-derived patches. Immunohistological analysis was used to characterize the composition of donor derived GFP⁺ green_patches. Imaris software was used to determine fluorescence co-localization. GFP⁺ areas were set as the area of interest, and the co-localization channel identified pixels that included both red (antibody staining) and green (donor derived cell labeling) fluorescence. Epithelial cells were stained with wide spectrum cytokeratin antibody (Figures 18A-C); endothelial cells were stained with anti-CD31 antibody (Figures 18D-F); and mesenchymal cells were stained with anti-Nestin antibody (Figures 18G-I). In each figure, the Red channel represents the lineage marker, the Green channel marks donor derived GFP⁺ cells, and the White channel denotes co-localization. Scale bar = 50 µm.

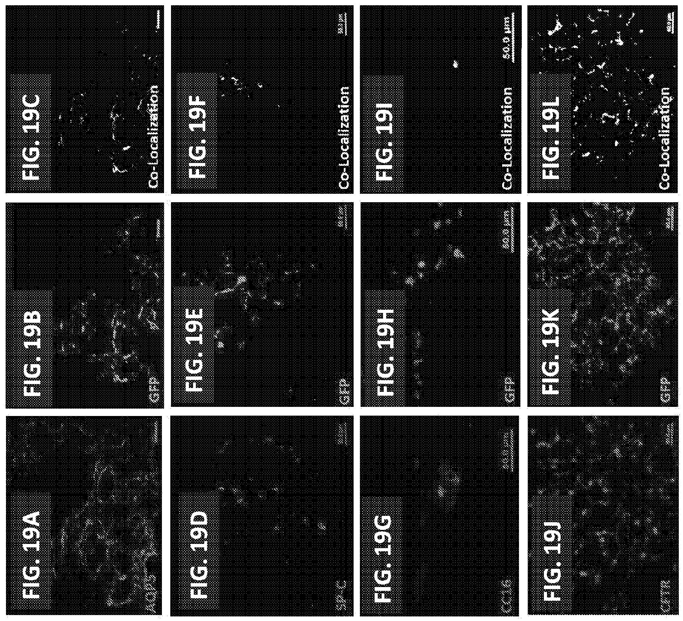

FIGs. 19A-L illustrate integration of donor-derived cells in the epithelial lung compartment. Immunohistological analysis was used to characterize the composition of the epithelial compartment within donor-derived GFP⁺ green patches. Imaris software was used to determine fluorescence co-localization. GFP⁺ areas were designated as the areas of interest, and the co-localization channel identified pixels that included both red and green colors. ATI cells were stained with anti-Aquaporin type 5 antibody (Figures 19A-C), ATII cells with anti- surfactant protein C antibody (Figures 19D-F), Club cells with anti-CCSP (CC16) (Figures 19G-I) antibody, and CFTR⁺ cells with anti-CFTR antibody (Figures 19J-L). In each figure, the Red channel represents the lineage marker, the Green channel marks donor-derived GFP⁺ cells, and the, and the White channel denotes co-localization. Scale bar = 50 µm.

### DESCRIPTION OF SPECIFIC EMBODIMENTS OF THE INVENTION

The present invention is defined in the claims.

The principles and operation of the present invention may be better understood with reference to the drawings and accompanying descriptions.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

While reducing the present invention to practice, the present inventors have surprisingly uncovered that the canalicular embryonic lung comprises epithelial lung progenitors as well as a marked level of hematopoietic progenitor cells (HPCs) which can induce durable and multi-lineage hematopoietic chimerism. Therefore, a successful transplantation of lung cells (in suspension) from a mis-matched allogeneic donor can be attained in the absence of chronic immunosuppression by transplantation of lung tissue cells comprising an effective amount of HPCs.

As is shown hereinbelow and in the Examples section which follows, the present inventors have uncovered through laborious experimentation that the canalicular embryonic lung comprises not only epithelial lung progenitors but also a marked level of HPCs which can induce durable and multi-lineage hematopoietic chimerism (Figures 1A-F). These lung hematopoietic progenitors can effectively compete with normal adult bone marrow stem cells (Figures 2A-D) and can be utilized in transplantation settings for induction of immune tolerance. The present inventors have further illustrated that fresh adult lung cells can induce both lung and blood chimerism (Figures 6A-F and Figures 7A-J, respectively) similar to that obtained by E16 fetal lung cells. Moreover, it was shown that *ex vivo* expanded fetal lung cells (E16) can be efficiently used for induction of lung chimerism (Figures 8A-F).

The present inventors established a new sub-lethal conditioning protocol for allogeneic lung transplantation comprising preconditioning the recipient animal with *in vivo* T cell debulking, naphthalene and total body irradiation (TBI). The recipients were then administered donor-derived single cell suspension of T cell depleted lung cells shortly followed by short-term immunosuppression with cyclophosphamide. This protocol attained a durable immune tolerance and lung chimerism for the allogeneic embryonic lung cells by virtue of the HSCs (Figures 4A-L and 5A-F).

The present inventors have further illustrated that adult lung cells can be used as an alternative source for fetal tissues for transplantation. Specifically, the present inventors have illustrated that donor-derived lung cells comprise hematopoietic, endothelial, epithelial and mesenchymal progenitors (Figures 16A-D) which differentiate into functional lung tissue after transplantation (Figure 15). Taken together, the present results illustrate that adult lung, obtained from cadaveric lungs or from live donors, can represent a suitable alternative source to fetal lung cells for repair of lung injury or disease in the absence of long-term immunosuppression.

Thus, according to one aspect of the present invention there is non-syngeneic pulmonary tissue cells in suspension comprising an effective amount of hematopoietic precursor cells (HPCs) or supplemented with HPCs, wherein the effective amount is a sufficient amount to achieve tolerance to the pulmonary tissue cells in the absence of chronic immunosuppressive regimen, and wherein said effective amount of said HPCs comprises at least about 1 x 10⁵ cells per Kg body weight of a subject, for use in treating a pulmonary disorder or injury in a subject in need thereof or for inducing donor specific tolerance in a subject in need of a pulmonary cell or tissue transplantation.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the term "subject" or "subject in need thereof' refers to a mammal, preferably a human being, male or female at any age that suffers from or is predisposed to a pulmonary tissue damage or deficiency as a result of a disease, disorder or injury. Typically the subject is in need of pulmonary cell or tissue transplantation (also referred to herein as recipient) due to a disorder or a pathological or undesired condition, state, or syndrome, or a physical, morphological or physiological abnormality which results in loss of organ functionality and is amenable to treatment via pulmonary cell or tissue transplantation.

As used herein, the phrase "pulmonary disorder or injury" refers to any disease, disorder, condition or to any pathological or undesired condition, state, or syndrome, or to any physical, morphological or physiological abnormality which involves a loss or deficiency of pulmonary cells or tissues or in loss-of-function of pulmonary cells or tissues.

Exemplary pulmonary diseases, include but are not limited to, cystic fibrosis (CF), emphysema, asbestosis, chronic obstructive pulmonary disease (COPD), pulmonary fibrosis, idiopatic pulmonary fibrosis, pulmonary hypertension, lung cancer, sarcoidosis, acute lung injury (adult respiratory distress syndrome), respiratory distress syndrome of prematurity, chronic lung disease of prematurity (bronchopulmonarydysplasia), surfactant protein B deficiency, congenital diaphragmatic hernia, pulmonary alveolar proteinosis, pulmonary hypoplasia, pneumonia (e.g. including that caused by bacteria, viruses, or fungi), asthma, idiopathic pulmonary fibrosis, nonspecific interstitial pneumonitis (e.g. including that present with autoimmune conditions, such as lupus, rheumatoid arthritis or scleroderma), hypersensitivity pneumonitis, cryptogenic organizing pneumonia (COP), acute interstitial pneumonitis, desquamative interstitial pneumonitis, asbestosis, and lung injury (e.g. induced by ischemia/reperfusion pulmonary hypertension or hyperoxic lung injury).

According to one embodiment, the pulmonary disorder or injury comprises chronic inflammation of the lungs (e.g. an inflammation lasting for more than two weeks).

Exemplary chronic inflammation conditions of the lungs include, but are not limited to, chronic airway inflammation, asthma, chronic obstructive pulmonary disease (COPD), lung cancer, cystic fibrosis (CF), granulomatous lung diseases, idiopatic pulmonary fibrosis, chronic lung disease of prematurity, radiation induced pneumonitis, lung diseases associated with systemic diseases such as scleroderma, lupus, dermatomyositis, sarcoidosis, and adult and neonatal respiratory distress syndrome.

According to one embodiment, the subject may benefit from transplantation of pulmonary cells or tissues.

The phrase "pulmonary tissue cells in suspension" as used herein refers to cells which have been isolated from their natural environment (e.g., the human body) are extracted from the pulmonary tissue while maintaining viability but do not maintain a tissue structure (i.e., no vascularized tissue structure) and are not attached to a solid support.

The phrase "pulmonary tissue" as used herein refers to a lung tissue or organ. The pulmonary tissue of the present invention may be a full or partial organ or tissue. Thus, the pulmonary tissue of the present invention may comprise the right lung, the left lung, or both. The pulmonary tissue of the present invention may comprise one, two, three, four or five lobes (from either the right or the left lung). Moreover, the pulmonary tissue of the present invention may comprise one or more lung segments or lung lobules. Furthermore, the pulmonary tissue of the present invention may comprise any number of bronchi and bronchioles (e.g. bronchial tree) and any number of alveoli or alveolar sacs.

Depending on the application, the method may be effected using pulmonary tissue cells which are syngeneic or non-syngeneic with the subject.

As used herein, the term "syngeneic" cells refer to cells which are essentially genetically identical with the subject or essentially all lymphocytes of the subject. Examples of syngeneic cells include cells derived from the subject (also referred to in the art as an "autologous"), from a clone of the subject, or from an identical twin of the subject.

As used herein, the term "non-syngeneic" cells refer to cells which are not essentially genetically identical with the subject or essentially all lymphocytes of the subject, such as allogeneic cells or xenogeneic cells.

As used herein, the term "allogeneic" refers to cells which are derived from a donor who is of the same species as the subject, but which is substantially non-clonal with the subject. Typically, outbred, non-zygotic twin mammals of the same species are allogeneic with each other. It will be appreciated that an allogeneic cell may be HLA identical, partially HLA identical or HLA non-identical (i.e. displaying one or more disparate HLA determinant) with respect to the subject.

As used herein, the term "xenogeneic" refers to a cell which substantially expresses antigens of a different species relative to the species of a substantial proportion of the lymphocytes of the subject. Typically, outbred mammals of different species are xenogeneic with each other.

The present invention envisages that xenogeneic cells are derived from a variety of species. Thus, according to one embodiment, the pulmonary tissue cells are derived from any mammal. Suitable species origins for the pulmonary tissue cells comprise the major domesticated or livestock animals and primates. Such animals include, but are not limited to, porcines (e.g. pig), bovines (e.g., cow), equines (e.g., horse), ovines (e.g., goat, sheep), felines (e.g., *Felis domestica),* canines (e.g., *Canis domestica),* rodents (e.g., mouse, rat, rabbit, guinea pig, gerbil, hamster), and primates (e.g., chimpanzee, rhesus monkey, macaque monkey, marmoset).

Pulmonary tissue cells of xenogeneic origin (e.g. porcine origin) are preferably obtained from a source which is known to be free of zoonoses, such as porcine endogenous retroviruses. Similarly, human-derived cells or tissues are preferably obtained from substantially pathogen-free sources.

According to one embodiment, the pulmonary tissue cells are non-syngeneic with the subject.

According to one embodiment, the pulmonary tissue cells are allogeneic with the subject.

According to one embodiment, the pulmonary tissue cells are xenogeneic with the subject.

According to an embodiment of the present invention, the subject is a human being and the pulmonary tissue cells are from a mammalian origin (e.g. allogeneic or xenogeneic).

According to an embodiment of the present invention, the subject is a human being and the pulmonary tissue cells are from a human origin (e.g. syngeneic or non-syngeneic).

According to one embodiment, the subject is a human being and the pulmonary tissue cells are from a xenogeneic origin (e.g. porcine origin).

Depending on the application and available sources, the cells for use according to the present invention may be obtained from a prenatal organism, postnatal organism, an adult or a cadaver donor. Such determinations are well within the ability of one of ordinary skill in the art.

It will be appreciated that the pulmonary tissue cells may be of fresh or frozen (e.g., cryopreserved) preparations, as further discussed below.

According to one embodiment, the pulmonary tissue cells are from an embryonic origin (e.g. corresponding to human gestation of one to eight weeks after fertilization).

According to one embodiment, the pulmonary tissue cells are from a fetal origin (e.g. corresponding to human gestation starting nine weeks after fertilization).

According to one embodiment, the pulmonary tissue cells are from an adult origin (e.g. a mammalian organism at any stage after birth).

Accordingly, the embryonic or fetal organism may be of any of a human or xenogeneic origin (e.g. porcine) and at any stage of gestation. Such a determination is in the capacity of one of ordinary skill in the art.

Various methods may be employed to obtain an organ or tissue from an embryonic or fetal organism. Thus, for example, obtaining a pulmonary tissue may be effected by harvesting the tissue from a developing fetus, e.g. by a surgical procedure.

According to one embodiment, the pulmonary tissue (i.e. lung tissue) is obtained from a fetus at a stage of gestation corresponding to human canalicular stage of development (e.g. 16-25 weeks of gestation). According to one embodiment, the pulmonary tissue is obtained from a fetus at a stage of gestation corresponding to human 16-17 weeks of gestation, 16-18 weeks of gestation, 16-19 weeks of gestation, 16-20 weeks of gestation, 16-21 weeks of gestation, 16-22 weeks of gestation, 16-24 weeks of gestation, 17-18 weeks of gestation, 17-19 weeks of gestation, 17-20 weeks of gestation, 17-21 weeks of gestation, 17-22 weeks of gestation, 17-24 weeks of gestation, 18-19 weeks of gestation, 18-20 weeks of gestation, 18-21 weeks of gestation, 18-22 weeks of gestation, 18-24 weeks of gestation, 19-20 weeks of gestation, 19-21 weeks of gestation, 19-22 weeks of gestation, 19-23 weeks of gestation, 19-24 weeks of gestation, 20-21 weeks of gestation, 20-22 weeks of gestation, 20-23 weeks of gestation, 20-24 weeks of gestation, 21-22 weeks of gestation, 21-23 weeks of gestation, 21-24 weeks of gestation, 22-23 weeks of gestation, 22-24 weeks of gestation, 22-25 weeks of gestation, 23-24 weeks of gestation, 23-25 weeks of gestation, 24-25 weeks of gestation or 25-26 weeks of gestation.

According to a specific embodiment, the pulmonary tissue is obtained from a fetus at a stage of gestation corresponding to human 20-22 weeks of gestation.

According to a specific embodiment, the pulmonary tissue is obtained from a fetus at a stage of gestation corresponding to human 21-22 days of gestation.

According to a specific embodiment, the pulmonary tissue is obtained from a fetus at a stage of gestation corresponding to human 20-21 days of gestation.

It will be understood by those of skill in the art that the gestational stage of an organism is the time period elapsed following fertilization of the oocyte generating the organism. The following table provides an example of the gestational stages of human and porcine tissues at which these can provide fetal tissues which are essentially at corresponding developmental stages:

**Table 1: Corresponding gestational stages of pigs and humans**

| **Gestational stage of porcine pulmonary tissue (days)** | **Gestational stage of human tissue (days*)** |
|---|---|
| 18 | 44 |
| 20 | 49 |
| 22 | 54 |
| 23 | 56-57 |
| 25 | 61-62 |
| 26 | 63 |
| 28 | 68-69 |
| 31 | 75 |
| 38 | 92 |
| 42 | 102 |
| 46 | 112 |
| 49 | 119 |
| 56 | 136 |
| 62 | 151 |
| 72 | 175 |
| 80 | 195 |
| 88 | 214 |

The gestational stage (in days) of a tissue belonging to a given species which is at a developmental stage essentially corresponding to that of a porcine tissue can be calculated according to the following formula: [gestational stage of porcine tissue in days] / [gestational period of pig in days] x [gestational stage of tissue of given species in days]. Similarly, the gestational stage (in days) of a tissue belonging to a given species which is at a developmental stage essentially corresponding to that of a human tissue can be calculated according to the following formula: [gestational stage of human tissue in days] / [gestational period of humans in days] x [gestational stage of tissue of given species in days]. The gestational stage of pigs is about 115 days and that of humans is about 280 days. * for week calculation divide the numbers by 7.

Likewise, various methods may be employed to obtain a pulmonary organ or tissue from an adult organism (e.g. live or cadaver). Thus, for example, obtaining a pulmonary tissue may be effected by harvesting the tissue from an organ donor by a surgical procedure e.g. laparotomy or laparoscopy. After the organ/tissue is obtained from the adult organism, pulmonary tissue cells as well as hematopoietic progenitor cells (as discussed in detail below) may be isolated therefrom according to methods known in the art, such methods depend on the source and lineage of the cells and may include, for example, flow cytometry and cell sorting as taught for example by www(dot)bio-rad(dot)com/en-uk/applications-technologies/isolation-maintenance-stem-cells.

It will be appreciated that in order to obtain pulmonary tissue cells, the pulmonary tissue need not be intact (i.e. maintain a tissue structure such that is suitable for a whole organ transplantation), however, the pulmonary tissue should comprise viable cells.

After a pulmonary organ/tissue is obtained (e.g. fetal or adult tissue), the present invention further contemplates generation of an isolated population of cells therefrom.

Thus, the pulmonary tissue cells may be comprised in a suspension of single cells or cell aggregates of no more than 5, 10, 50, 100, 200, 300, 400, 500, 1000, 1500, 2000 cells in an aggregate.

The cell suspension of the invention may be obtained by any mechanical or chemical (e.g. enzymatic) means. Several methods exist for dissociating cell clusters to form cell suspensions (e.g. single cell suspension) from primary tissues, attached cells in culture, and aggregates, e.g., physical forces (mechanical dissociation such as cell scraper, trituration through a narrow bore pipette, fine needle aspiration, vortex disaggregation and forced filtration through a fine nylon or stainless steel mesh), enzymes (enzymatic dissociation such as trypsin, collagenase, Acutase and the like) or a combination of both.

Thus, for example, enzymatic digestion of tissue/organ into isolate cells can be performed by subjecting the tissue to an enzyme such as type IV Collagenase (Worthington biochemical corporation, Lakewood, NJ, USA) and/or Dispase (Invitrogen Corporation products, Grand Island NY, USA). For example, the tissue may be enzyme digested by finely mincing tissue with a razor blade in the presence of e.g. collagenase, dispase and CaCl₂ at 37 °C for about 1 hour. The method may further comprise removal of nonspecific debris from the resultant cell suspension by, for example, sequential filtration through filters (e.g. 70- and 40-µm filters), essentially as described under "General Materials and Experimental Methods" of the Examples section which follows.

Furthermore, mechanical dissociation of tissue into isolated cells can be performed using a device designed to break the tissue to a predetermined size. Such a device can be obtained from CellArtis Goteborg, Sweden. Additionally or alternatively, mechanical dissociation can be manually performed using a needle such as a 27g needle (BD Microlance, Drogheda, Ireland) while viewing the tissue/cells under an inverted microscope.

Following enzymatic or mechanical dissociation of the tissue, the dissociated cells are further broken to small clumps using 200 µl Gilson pipette tips (e.g., by pipetting up and down the cells).

According to one embodiment, the cells for use according to the present invention may be genetically modified prior to transplantation.

A pulmonary tissue at a desired developmental stage may also be obtained by *in-vitro* culture of cells, organs/tissues. Such controlled *in-vitro* differentiation of cells, tissues or organs is routinely performed, for example, using culturing of embryonic stem cell lines to generate cultures containing cells/tissues/organs of desired lineages. For example, for generation of pulmonary lineages, refer for example, to Otto WR., 1997. Int J Exp Pathol. 78:291-310.

According to one embodiment, the cells are *ex-vivo* differentiated from adult stem cells or pluripotent stem cells (e.g. de-differentiated), such as embryonic stem cells (ES cells) or iPS.

The phrase "embryonic stem cells" or "ES cells" refers to embryonic cells which are capable of differentiating into cells of all three embryonic germ layers *(i.e*., endoderm, ectoderm and mesoderm), or remaining in an undifferentiated state. The phrase "embryonic stem cells" may comprise cells which are obtained from the embryonic tissue formed after gestation (e.g., blastocyst) before implantation of the embryo (*i.e*., a pre-implantation blastocyst), extended blastocyst cells (EBCs) which are obtained from a post-implantation/pre-gastrulation stage blastocyst (see WO2006/040763), embryonic germ (EG) cells which are obtained from the genital tissue of a fetus any time during gestation, preferably before 10 weeks of gestation, and cells originating from an unfertilized ova which are stimulated by parthenogenesis (parthenotes).

Embryonic stem cells (e.g., human ESCs) originating from an unfertilized ova stimulated by parthenogenesis (parthenotes) are known in the art (e.g., Zhenyu Lu et al., 2010. J. Assist Reprod. Genet. 27:285-291; "Derivation and long-term culture of human parthenogenetic embryonic stem cells using human foreskin feeders", which is fully incorporated herein by reference). Parthenogenesis refers to the initiation of cell division by activation of ova in the absence of sperm cells, for example using electrical or chemical stimulation. The activated ovum (parthenote) is capable of developing into a primitive embryonic structure (called a blastocyst) but cannot develop to term as the cells are pluripotent, meaning that they cannot develop the necessary extra-embryonic tissues (such as amniotic fluid) needed for a viable human fetus.

Another method for preparing ES cells is described in Chung et al., Cell Stem Cell, Volume 2, Issue 2, 113-117, 7 February 2008. This method comprises removing a single cell from an embryo during an *in vitro* fertilization process. The embryo is not destroyed in this process.

Induced pluripotent stem cells (iPS; embryonic-like stem cells), are cells obtained by de-differentiation of adult somatic cells which are endowed with pluripotency *(i.e.,* being capable of differentiating into the three embryonic germ cell layers, *i.e.,* endoderm, ectoderm and mesoderm). According to some embodiments of the invention, such cells are obtained from a differentiated tissue (e.g., a somatic tissue such as skin) and undergo de-differentiation by genetic manipulation, which re-program the cell to acquire embryonic stem cells characteristics. According to some embodiments of the invention, the induced pluripotent stem cells are formed by inducing the expression of Oct-4, Sox2, Kfl4 and/or c-Myc in a somatic stem cell.

According to one embodiment, the pluripotent stem cells are not a result of embryo destruction.

The phrase "adult stem cells" (also called "tissue stem cells" or a stem cell from a somatic tissue) refers to any stem cell derived from a somatic tissue [of either a postnatal or prenatal animal (especially the human)]. The adult stem cell is generally thought to be a multipotent stem cell, capable of differentiation into multiple cell types. Adult stem cells can be derived from any adult, neonatal or fetal tissue such as adipose tissue, skin, kidney, liver, prostate, pancreas, intestine, bone marrow and placenta.

Cultured embryonic stem cells can be differentiated into restricted developmental lineage cells (e.g. pulmonary tissue cells).

Differentiation of stem cells can be initiated by allowing overgrowth of undifferentiated human ES cells in suspension culture forming embryoid bodies or by plating ES cells under conditions that promote differentiation in a particular manner.

It will be appreciated that the culturing conditions suitable for the differentiation and expansion of the isolated lineage specific cells include various tissue culture medium, growth factors, antibiotic, amino acids and the like and it is within the capability of one skilled in the art to determine which conditions should be applied in order to expand and differentiate particular cell types and/or cell lineages [reviewed in Fijnvandraat AC, et al., Cardiovasc Res. 2003; 58: 303-12; Sachinidis A, et al., Cardiovasc Res. 2003; 58: 278-91; Stavridis MP and Smith AG, 2003; Biochem Soc Trans. 31(Pt 1): 45-9].

Human pluripotent stem cells have also been induced to differentiate into lung and airway progenitor cells [Huang et al., Nature Protocols 10, 413-425 (2015)]. Thus, the stem cells can be cultured in the presence of high concentrations of activin A. Subsequently, lung-biased anterior foregut endoderm (AFE) is specified by sequential inhibition of bone morphogenetic protein (BMP), transforming growth factor-β (TGF-β) and Wnt signaling. AFE is then ventralized by applying Wnt, BMP, fibroblast growth factor (FGF) and retinoic acid (RA) signaling to obtain lung and airway progenitors.

Differentiation of stem cells can also be directed by genetic modification. For example expression of four factors GATA-4 TBX5, NKX2.5 and BAF60c was shown to induce differentiation of hESCs into cardiac progenitors [Dixon et al., Molecular Therapy (2011) 19 9, 1695-1703].

### Monitoring the differentiation stage of embryonic stem cells

During the culturing step the stem cells are further monitored for their differentiation state. Cell differentiation can be determined upon examination of cell or tissue-specific markers which are known to be indicative of differentiation. For example, primate ES cells may express the stage-specific embryonic antigen (SSEA) 4, the tumor-rejecting antigen (TRA)-1-60 and TRA-1-81.

Tissue/cell specific markers can be detected using immunological techniques well known in the art [Thomson JA et al., (1998). Science 282: 1145-7]. Examples include, but are not limited to, flow cytometry for membrane-bound markers, immunohistochemistry for extracellular and intracellular markers and enzymatic immunoassay, for secreted molecular markers.

Determination of ES cell differentiation can also be effected via measurements of alkaline phosphatase activity. Undifferentiated human ES cells have alkaline phosphatase activity which can be detected by fixing the cells with 4 % paraformaldehyde and developing with the Vector Red substrate kit according to manufacturer's instructions (Vector Laboratories, Burlingame, California, USA).

The pluripotency of embryonic stem cells can be monitored *in vitro* by the formation of embryoid bodies (EBs) as well as *in vivo* via the formation of teratomas.

### Teratomas

The pluripotent capacity of the ES cell line can also be confirmed by injecting cells into SCID mice [Evans MJ and Kaufman M (1983). Pluripotential cells grown directly from normal mouse embryos. Cancer Surv. 2: 185-208], which upon injection form teratomas. Teratomas are fixed using 4 % paraformaldehyde and histologically examined for the three germ layers *(i.e.,* endoderm, mesoderm and ectoderm).

In addition to monitoring a differentiation state, stem cells are often also being monitored for karyotype, in order to verify cytological euploidity, wherein all chromosomes are present and not detectably altered during culturing. Cultured stem cells can be karyotyped using a standard Giemsa staining and compared to published karyotypes of the corresponding species.

According to the present invention, the cell suspension of pulmonary tissue cells comprises viable cells. Cell viability may be monitored using any method known in the art, as for example, using a cell viability assay (e.g. MultiTox Multiplex Assay available from Promega), Flow cytometry, Trypan blue, etc.

Typically, the pulmonary tissue cells are immediately used for transplantation. However, in situations in which the cells are to be maintained in suspension prior to transplantation, e.g. for 1-12 hours, the cells may be cultured in a culture medium which is capable of supporting their viability. Such a culture medium can be a water-based medium which includes a combination of substances such as salts, nutrients, minerals, vitamins, amino acids, nucleic acids, proteins such as cytokines, growth factors and hormones, all of which are needed for maintaining the pulmonary tissue cells in an viable state. For example, a culture medium according to this aspect of the present invention can be a synthetic tissue culture medium such as RPMI-1640 (Life Technologies, Israel), Ko-DMEM (Gibco-Invitrogen Corporation products, Grand Island, NY, USA), DMEM/F12 (Biological Industries, Beit Haemek, Israel), Mab ADCB medium (HyClone, Utah, USA) or DMEM/F12 (Biological Industries, Biet Haemek, Israel) supplemented with the necessary additives. Preferably, all ingredients included in the culture medium of the present invention are substantially pure, with a tissue culture grade.

The pulmonary tissue cells may also be stored under appropriate conditions (typically by freezing) to keep the cells (e.g. pulmonary tissue cells) alive and functioning for use in transplantation. According to one embodiment, the pulmonary tissue cells are stored as cryopreserved populations. Other preservation methods are described in U.S. Pat. Nos. 5,656,498, 5,004,681, 5,192,553, 5,955,257, and 6,461,645. Methods for banking stem cells are described, for example, in U.S. Patent Application Publication No. 2003/0215942.

The pulmonary tissue cells may also be expanded, e.g. *ex vivo.* The term "expanded" refers to increasing the cell number by way of proliferation by at least about 2 fold, 4 fold, 10 fold, 20 fold, 40 fold, 80 fold, 120 fold, by 140 fold or more over a given time interval (and as compared to non-expanded cells).

According to one embodiment, the pulmonary tissue cells are expanded in culture (e.g. *ex-vivo* expanded) from adult cells (e.g. adult pulmonary cells).

According to one embodiment, the pulmonary tissue cells are expanded in culture (e.g. *ex-vivo* expanded) from fetal cells (e.g. fetal pulmonary cells). The fetal cells may be of a gestational age as discussed above (e.g. 14-22 weeks, e.g. 15-16 weeks, of human gestation).

Thus, for example, the pulmonary tissue cells may be obtained from a fetal tissue (e.g. fetal lung tissue) and cultured in tissue culture plates in the presence of a cell medium (e.g. feeder medium, e.g. iMEF) and optionally with additional growth factors and/or cytokines (e.g. epithelial growth factor and Rho-associated kinase (ROCK) inhibitor) for several days (e.g. 7-14 days, such as 9 days), until a suitable number of cells is obtained. Measuring the number of cells (e.g. viable cells) can be carried out using any method known to one of skill in the art, e.g. by a counting chamber, by FACs analysis, or by a spectrophotometer. Further protocols for cell culture and expansion can be found for example in Liu X. et al., Am J Pathol. (2012) 180(2): 599-607; Palechor-Ceron N et al, Am J Pathol. (2013) 183(6): 1862-70; Zhang L. et al., PLoS One. (2011) 6(3):e18271; Terunuma A. et al., Tissue Eng Part A. (2010) 16(4):1363-8; and Bhandary L. et al., Oncotarget. (2015) 6(8):6251-66, all of which are incorporated herein by reference.

The pulmonary tissue cells may comprise cells obtained from more than one cell donor.

According to one embodiment, the pulmonary tissue cells are depleted of T cells.

As used herein the phrase "depleted of T cells" refers to a population of pulmonary tissue cells which are depleted of T lymphocytes. The T cell depleted pulmonary tissue cells may be depleted of CD3⁺ cells, CD2⁺ cells, CD8⁺ cells, CD4⁺ cells, α/β T cells and/or γ/δ T cells.

According to one embodiment, the pulmonary tissue cells are depleted of CD3⁺ T cells.

According to an embodiment, the T cell depleted pulmonary tissue cells comprise less than 50 x 10⁵ CD3⁺ T cells, 40 x 10⁵ CD3⁺ T cells, 30 x 10⁵ CD3⁺ T cells, 20 x 10⁵ CD3⁺ T cells, 15 x 10⁵ CD3⁺ T cells, 10 x 10⁵ CD3⁺ T cells, 9 x 10⁵ CD3⁺ T cells, 8 x 10⁵ CD3⁺ T cells, 7 x 10⁵ CD3⁺ T cells, 6 x 10⁵ CD3⁺ T cells, 5 x 10⁵ CD3⁺ T cells, 4 x 10⁵ CD3⁺ T cells, 3 x 10⁵ CD3⁺ T cells, 2 x 10⁵ CD3⁺ T cells, 1 x 10⁵ CD3⁺ T cells or 5 x 10⁴ CD3⁺ T cells per kilogram body weight of the subject.

According to a specific embodiment, the T cell depleted pulmonary tissue cells comprise less than 1 x 10⁵ CD3⁺ T cells per kilogram body weight of the subject.

According to one embodiment, the pulmonary tissue cells are depleted of CD8⁺ cells.

According to an embodiment, the T cell depleted pulmonary tissue cells comprise less than 50 x 10⁵ CD8⁺ cells, 25 x 10⁵ CD8⁺ cells, 15 x 10⁵ CD8⁺ cells, 10 x 10⁵ CD8⁺ cells, 9 x 10⁵ CD8⁺ cells, 8 x 10⁵ CD8⁺ cells, 7 x 10⁵ CD8⁺ cells, 6 x 10⁵ CD8⁺ cells, 5 x 10⁵ CD8⁺ cells, 4 x 10⁵ CD8⁺ cells, 3 x 10⁵ CD8⁺ cells, 2 x 10⁵ CD8⁺ cells, 1 x 10⁵ CD8⁺ cells, 9 x 10⁴ CD8⁺ cells, 8 x 10⁴ CD8⁺ cells, 7 x 10⁴ CD8⁺ cells, 6 x 10⁴ CD8⁺ cells, 5 x 10⁴ CD8⁺ cells, 4 x 10⁴ CD8⁺ cells, 3 x 10⁴ CD8⁺ cells, 2 x 10⁴ CD8⁺ cells or 1 x 10⁴ CD8⁺ cells per kilogram body weight of the subject.

According to one embodiment, the pulmonary tissue cells are depleted of B cells.

According to an embodiment, the pulmonary tissue cells are depleted of B cells (CD19⁺ and/or CD20⁺ B cells).

According to an embodiment, the pulmonary tissue cells comprise less than 50 x 10⁵ B cells, 40 x 10⁵ B cells, 30 x 10⁵ B cells, 20 x 10⁵ B cells, 10 x 10⁵ B cells, 9 x 10⁵ B cells, 8 x 10⁵ B cells, 7 x 10⁵ B cells, 6 x 10⁵ B cells, 5 x 10⁵ B cells, 4 x 10⁵ B cells, 3 x 10⁵ B cells, 2 x 10⁵ B cells or 1 x 10⁵ B cells per kilogram body weight of the subject.

Depletion of T cells, e.g. CD3⁺, CD2⁺, TCRα/β⁺, CD4⁺ and/or CD8⁺ cells, or B cells, e.g. CD19+ and/or CD20⁺ cells, may be carried out using any method known in the art, such as by eradication (e.g. killing) with specific antibodies or by affinity based purification e.g. such as by the use of magnetic cell separation techniques, FACS sorter and/or capture ELISA labeling.

Such methods are described herein and in THE HANDBOOK OF EXPERIMENTAL IMMUNOLOGY, Volumes 1 to 4, (D.N. Weir, editor) and FLOW CYTOMETRY AND CELL SORTING (A. Radbruch, editor, Springer Verlag, 1992). For example, cells can be sorted by, for example, flow cytometry or FACS. Thus, fluorescence activated cell sorting (FACS) may be used and may have varying degrees of color channels, low angle and obtuse light scattering detecting channels, and impedance channels. Any ligand-dependent separation techniques known in the art may be used in conjunction with both positive and negative separation techniques that rely on the physical properties of the cells rather than antibody affinity, including but not limited to elutriation and density gradient centrifugation.

Other methods for cell sorting include, for example, panning and separation using affinity techniques, including those techniques using solid supports such as plates, beads and columns. Thus, biological samples may be separated by "panning" with an antibody attached to a solid matrix, e.g. to a plate.

Alternatively, cells may be sorted/separated by magnetic separation techniques, and some of these methods utilize magnetic beads. Different magnetic beads are available from a number of sources, including for example, Dynal (Norway), Advanced Magnetics (Cambridge, MA, U.S.A.), Immuncon (Philadelphia, U.S.A.), Immunotec (Marseille, France), Invitrogen, Stem cell Technologies (U.S.A) and Cellpro (U.S.A). Alternatively, antibodies can be biotinylated or conjugated with digoxigenin and used in conjunction with avidin or anti-digoxigenin coated affinity columns.

According to an embodiment, different depletion/separation methods can be combined, for example, magnetic cell sorting can be combined with FACS, to increase the separation quality or to allow sorting by multiple parameters.

According to one embodiment, the T cell depleted pulmonary tissue cells are obtained by T cell debulking (TCD).

T cell debulking may be effected using antibodies, including e.g. anti-CD8 antibodies, anti-CD4 antibodies, anti-CD3 antibodies, anti-CD2 antibodies, anti-TCRα/β antibodies and/or anti-TCRγ/δ antibodies.

According to one embodiment, depletion of B cells is effected by B cell debulking.

B cell debulking may be effected using antibodies, including e.g. anti-CD 19 or anti-CD20 antibodies. Alternatively, debulking *in-vivo* of B cells can be attained by infusion of anti-CD20 antibodies.

T cell or B cell debulking may be effected *in-vitro* or *in-vivo* (e.g. in a donor prior to acquiring pulmonary tissue cells therefrom).

The pulmonary tissue cells for use according to the invention typically comprise a heterogeneous population of cells.

Thus, the pulmonary tissue cells may comprise any of hematopoietic progenitor cells, epithelial progenitor cells, mesenchymal progenitor cells and/or endothelial progenitor cells.

According to one embodiment, the cells comprise a cytokeratin 5⁺ (CK5⁺) marker expression.

According to one embodiment, the cells comprise a cytokeratin 5⁺ (CK5⁺) and cytokeratin 14⁺ (CK14⁺) marker expression.

According to one embodiment, the cells comprise a c-Kit⁺ CD45⁻ CD34⁻ marker expression.

According to one embodiment, the cells comprise a c-Kit⁺ CD45⁻ CD34⁻ CD31⁻ CD326⁻ CD271⁻ marker expression.

According to one embodiment, the cells comprise a c-Kit⁺ CD34⁺ marker expression.

According to one embodiment, the cells comprise a c-Kit⁺ CD34⁺ CD31⁺ marker expression.

According to one embodiment, the cells comprise a c-Kit⁺ CD34⁺ CD326⁺ marker expression.

According to one embodiment, the cells comprise a CD34⁺ CD31⁺ CD14⁺ CD45⁺ marker expression.

According to one embodiment, the cells comprise a CD34⁺ CD31⁺ CD45⁻ CD105⁺ marker expression.

According to one embodiment, the cells comprise CD31⁺ marker expression (e.g. the cells are endothelial progenitor cells).

According to one embodiment, the cells comprise a CD31⁺ CD144⁺ marker expression.

According to one embodiment, the cells comprise a CD31⁺ CD146⁺ marker expression.

According to one embodiment, the cells comprise CK⁺ marker expression (e.g. the cells are epithelial progenitor cells).

According to one embodiment, the cells comprise a SOX9⁺ marker expression.

According to one embodiment, the cells comprise a SOX2⁺ marker expression.

According to one embodiment, the cells comprise a NKX2.1 marker expression.

According to one embodiment, the cells comprise a Mucin⁺ Podoplanin ⁺ marker expression.

According to one embodiment, the cells comprise Epcam⁺ CD24⁺ SCA1⁺ marker expression.

According to one embodiment, the cells comprise Epcam⁺ CD24⁺ SCAl⁻ marker expression.

According to one embodiment, the cells comprise Epcam⁺ CD24⁺ Podoplanin⁺ Scal⁺ marker expression.

According to one embodiment, the cells comprise a nestin⁺ marker expression (e.g. the cells are mesenchymal progenitor cells).

According to one embodiment, the cells comprise a calcitonin gene related protein⁺ (CGRP⁺) marker expression.

According to one embodiment, the cells comprise an alpha smooth muscle actin⁺ (alpha-SMA⁺) marker expression.

According to one embodiment, the cells comprise a Vimentin⁺ marker expression.

According to a specific embodiment, the cells are hematopoietic progenitor cells.

According to one embodiment, the cells comprise CD45⁺ marker expression (e.g. hematopoietic progenitor cells).

According to a specific embodiment, the hematopoietic progenitor cells are LSK cells comprising a Lin⁻ Sca-1⁺ c-kit⁺ marker expression.

According to a specific embodiment, the hematopoietic progenitor cells are signaling lymphocytic activation molecule (SLAM) cells comprising a lineage negative CD41⁻ CD48⁻ CD150⁺ marker expression.

According to one embodiment, each of the cell populations mentioned hereinabove may be purified from the pulmonary tissue. According to a specific embodiment, each of the cell populations mentioned hereinabove may be of about 50 %, 60 %, 70 %, 80 %, 90 % or 100 % purification.

Purification of specific cell types may be carried out by any method known to one of skill in the art, as for example, by affinity based purification (e.g. such as by the use of MACS beads, FACS sorter and/or capture ELISA labeling) using specific antibodies which recognize any of the above described cell markers (e.g. CK5, CK14, c-Kit, Lin, Sca-1, CD31, CD34, CD41, CD45, CD48, CD105, CD150, CD271, CD326, etc.).

According to an embodiment of the present invention, the cell suspension comprises a non-purified mixture of the pulmonary tissue cells.

According to another embodiment, the pulmonary tissue cells in suspension comprise a cell-type specific population of pulmonary cells (e.g. pulmonary tissue cells depleted of T cells and/or HPCs). Isolating such cells may be carried out by any method known to one of skill in the art, as for example, by affinity based purification (e.g. such as by the use of MACS beads, FACS sorter and/or capture ELISA labeling, as mentioned above) or by eradication (e.g. killing) of unwanted cells with specific antibodies targeting same.

According to a specific embodiment, the pulmonary tissue cells in suspension comprise an effective amount of hematopoietic precursor cells (HPCs).

As used herein, the term "hematopoietic precursor cells" or "HPCs" refers to a cell preparation comprising immature hematopoietic cells. Such cell preparation includes or is derived from a biological sample, for example, pulmonary tissue (e.g. fetal or adult tissue), bone marrow (e.g. T cell depleted bone marrow), mobilized peripheral blood (e.g. mobilization of CD34⁺ cells to enhance their concentration), cord blood (e.g. umbilical cord), fetal liver, yolk sac and/or placenta. Additionally or alternatively, purified CD34⁺ cells or other hematopoietic stem cells, such as CD131⁺ cells, can be used in accordance with some embodiments of the present teachings, either with or without *ex-vivo* expansion.

As used herein, the term "an effective amount" refers to an amount sufficient to achieve tolerance to the pulmonary tissue cells in the absence of chronic immunosuppressive regimen.

As used herein, the term "tolerance" refers to a condition in which there is a decreased responsiveness of the recipient's cells (e.g. recipient's T cells) when they come in contact with the donor's cells (e.g. donor HPCs) as compared to the responsiveness of the recipient's cells in the absence of such a treatment method.

Tolerance induction enables transplantation of a cell or tissue graft (e.g. pulmonary tissue cells) with reduced risk of graft rejection or graft versus host disease (GVHD).

Without being bound to theory, tolerance towards the donor HPCs, in the recipient, leads to central tolerance (e.g. by negative selection in the thymus), resulting in tolerance towards the pulmonary tissue cells and achievement of a chimeric tissue (i.e. a tissue comprising cells from both the donor and the recipient).

As mentioned, the HPCs induce donor specific tolerance and overcome the need to use a chronic immunosuppressive regimen.

As used herein, the term "chronic immunosuppressive regimen" refers to administration immunosuppressive therapy for a prolonged period of time following transplantation (i.e. post transplantation), e.g. for more than 2 weeks, one month, two months, 6 months, 12 months, 2 years, 5 years or more.

An effective amount of HPCs typically comprise at least about 1 x 10⁵ - 10 x 10⁷ cells per Kg body weight of the subject.

According to one embodiment, the HPCs comprise a dose range of about 1-5 x 10⁵, 1-10 x 10⁵, 1-50 x 10⁵, 1-100 x 10⁵, 5-10 x 10⁵, 5-20 x 10⁵, 5-30 x 10⁵, 5-40 x 10⁵, 5-50 x 10⁵, 5-60 x 10⁵, 5-70 x 10⁵, 5-80 x 10⁵, 5-90 x 10⁵, 5-100 x 10⁵, 10-20 x 10⁵, 10-30 x 10⁵, 10-40 x 10⁵, 10-50 x 10⁵, 10-60 x 10⁵, 10-70 x 10⁵, 10-80 x 10⁵, 10-90 x 10⁵, 10-100 x 10⁵, 50-60 x 10⁵, 50-70 x 10⁵, 50-80 x 10⁵, 50-90 x 10⁵, 50-100 x 10⁵, 1-5 x 10⁶, 1-10 x 10⁶, 1-50 x 10⁶, 1-100 x 10⁶, 5-10 x 10⁶, 5-20 x 10⁶, 5-30 x 10⁶, 5-40 x 10⁶, 5-50 x 10⁶, 5-60 x 10⁶, 5-70 x 10⁶, 5-80 x 10⁶, 5-90 x 10⁶, 5-100 x 10⁶, 10-20 x 10⁶, 10-30 x 10⁶, 10-40 x 10⁶, 10-50 x 10⁶, 10-60 x 10⁶, 10-70 x 10⁶, 10-80 x 10⁶, 10-90 x 10⁶, 10-100 x 10⁶, 50-60 x 10⁶, 50-70 x 10⁶, 50-80 x 10⁶, 50-90 x 10⁶, 50-100 x 10⁶, 1-5 x 10⁷, 1-10 x 10⁷, 1-50 x 10⁷, 1-100 x 10⁷ 5-10 x 10⁷, 5-20 x 10⁷, 5-30 x 10⁷, 5-40 x 10⁷, 5-50 x 10⁷, 5-60 x 10⁷, 5-70 x 10⁷, 5-80 x 10⁷, 5-90 x 10⁷, 5-100 x 10⁷, 10-20 x 10⁷, 10-30 x 10⁷, 10-40 x 10⁷, 10-50 x 10⁷, 10-60 x 10⁷, 10-70 x 10⁷, 10-80 x 10⁷, 10-90 x 10⁷, 10-100 x 10⁷ 50-60 x 10⁷, 50-70 x 10⁷, 50-80 x 10⁷, 50-90 x 10⁷ or 50-100 x 10⁷ cells per Kg body weight of the subject.

According to a specific embodiment the HPCs comprise a dose range of about 1-10 x 10⁶ cells per Kg body weight of the subject.

According to a specific embodiment the HPCs comprise a dose range of about 5-30 x 10⁶ cells per Kg body weight of the subject.

According to one embodiment, the HPCs comprise at least about 1 x 10⁵, 2 x 10⁵, 3 x 10⁵, 4 x 10⁵, 5 x 10⁵, 6 x 10⁵, 7 x 10⁵, 8 x 10⁵, 9 x 10⁵, 10 x 10⁵, 1 x 10⁶, 2 x 10⁶, 3 x 10⁶, 4 x 10⁶, 5 x 10⁶, 6 x 10⁶, 7 x 10⁶, 8 x 10⁶, 9 x 10⁶, 10 x 10⁶, 1 x 10⁷, 2 x 10⁷, 3 x 10⁷, 4 x 10⁷, 5 x 10⁷, 6 x 10⁷, 7 x 10⁷, 8 x 10⁷, 9 x 10⁷, 10 x 10⁷, 1 x 10⁸, 2 x 10⁸, 3 x 10⁸, 4 x 10⁸, 5 x 10⁸, 6 x 10⁸, 7 x 10⁸, 8 x 10⁸, 9 x 10⁸ or 10 x 10⁸ cells per Kg body weight of the subject.

According to a specific embodiment, the HPCs comprise at least about 0.1 x 10⁶ cells per Kg body weight of the subject.

According to a specific embodiment, the HPCs comprise at least about 1 x 10⁶ cells per Kg body weight of the subject.

According to a specific embodiment, the HPCs comprise at least about 5 x 10⁶ cells per Kg body weight of the subject.

According to a specific embodiment, the HPCs comprise at least about 10 x 10⁶ cells per Kg body weight of the subject.

According to one embodiment, the HPCs comprise at least about 0.01-0.5 % of the pulmonary tissue cells in suspension.

According to one embodiment, the HPCs comprise at least about 0.01-1 % of the pulmonary tissue cells in suspension.

According to one embodiment, the HPCs comprise at least about 0.01-10 % of pulmonary tissue cells in suspension.

To augment the tolerance, the pulmonary tissue cells in suspension may be supplemented with HPCs.

According to one embodiment, the HPCs and the pulmonary tissue cells are obtained from the same tissue (i.e. from a pulmonary tissue of a fetus or adult donor).

According to one embodiment, the HPCs and the pulmonary tissue cells are obtained from the same donor (e.g. non-syngeneic donor).

According to one embodiment, the HPCs and the pulmonary tissue cells are obtained from different donors (e.g. from two non-syngeneic donors sharing HLA identity).

According to one embodiment, the pulmonary tissue cells and the HPCs are administered in the same formulation.

According to another embodiment, the pulmonary tissue cells and the HPCs are administered in separate formulation.

In cases where separate formulations are administered, the HPCs may be administered prior to, concomitantly with, or following administration of the pulmonary tissue cells.

Accordingly, the HPCs and pulmonary tissue cells may be administered together, on the same day, on subsequent days or even a few days apart from each other (e.g. 2, 3, 4, 5, 6, 7, 8, 9, 10 or 14 days apart).

Administration of the pulmonary tissue cells in suspension and/or HPCs to the subject may be effected in numerous ways, depending on various parameters, such as, for example, the type, stage or severity of the disease to be treated, the physical or physiological parameters specific to the individual subject, and/or the desired therapeutic outcome. For example, depending on the application and purpose administration of the pulmonary tissue cells in suspension and/or HPCs may be effected by a route selected from the group consisting of intratracheal, intrabronchial, intraalveolar, intravenous, intraperitoneal, intranasal, subcutaneous, intramedullary, intrathecal, intraventricular, intracardiac, intramuscular, intraserosal, intramucosal, transmucosal, transnasal, rectal and intestinal.

According to one embodiment, administering is effected by an intravenous route.

According to one embodiment, administering is effected by an intratracheal route.

Alternatively, administration of the pulmonary tissue cells to the subject may be effected by administration thereof into various suitable anatomical locations so as to be of therapeutic effect. Thus, depending on the application and purpose, the pulmonary tissue cells may be administered into a homotopic anatomical location (a normal anatomical location for the organ or tissue type of the cells), or into an ectopic anatomical location (an abnormal anatomical location for the organ or tissue type of the cells).

Accordingly, depending on the application and purpose, the pulmonary tissue cells may be advantageously implanted (e.g. transplanted) under the renal capsule, or into the kidney, the testicular fat, the sub cutis, the omentum, the portal vein, the liver, the spleen, the heart cavity, the heart, the chest cavity, the lung, the pancreas, the skin and/or the intra-abdominal space.

According to an embodiment of the present invention, the pulmonary tissue cells in suspension are administered to the subject at a dose range of about 1-10x 10⁶, 5-10x 10⁶, 1-50 x 10⁶, 10-50 x 10⁶, 10-60 x 10⁶, 10-70 x 10⁶, 10-80 x 10⁶, 10-90 x 10⁶, 1-100 x 10⁶, 5-100 x 10⁶, 10-100 x 10⁶, 50-100 x 10⁶, 1-200 x 10⁶, 5-200 x 10⁶, 10-200 x 10⁶, 50-200 x 10⁶, 100-200 x 10⁶, 1-500 x 10⁶, 5-500 x 10⁶, 10-500 x 10⁶, 100-500 x 10⁶, 1-1000 x 10⁶, 5-1000 x 10⁶, 10-1000 x 10⁶, 50-1000 x 10⁶, 100-1000 x 10⁶, 500-1000 x 10⁶, 1-2000 x 10⁶, 5-2000 x 10⁶, 10-2000 x 10⁶, 20-2000 x 10⁶, 30-2000 x 10⁶, 40-2000 x 10⁶, 50-2000 x 10⁶, 60-2000 x 10⁶, 70-2000 x 10⁶, 80-2000 x 10⁶, 90-2000 x 10⁶, 100-2000 x 10⁶, 200-2000 x 10⁶, 300-2000 x 10⁶, 400-2000 x 10⁶, 500-2000 x 10⁶, 600-2000 x 10⁶, 700-2000 x 10⁶, 800-2000 x 10⁶, 900-2000 x 10⁶, 1000-2000 x 10⁶, 1500-2000 x 10⁶, 100-3000 x 10⁶, 200-3000 x 10⁶, 300-3000 x 10⁶, 400-3000 x 10⁶, 500-3000 x 10⁶, 600-3000 x 10⁶, 700-3000 x 10⁶, 800-3000 x 10⁶, 900-3000 x 10⁶, 1000-3000 x 10⁶, 2000-3000 x 10⁶, 500-4000 x 10⁶, 1000-4000 x 10⁶, 2000-4000 x 10⁶, 3000-4000 x 10⁶ cells per Kg body weight of the subject.

According a specific embodiment, the pulmonary tissue cells in suspension are administered to the subject at a dose range of about 40-2000 x 10⁶ cells per Kg body weight of the subject (e.g. for administration of pulmonary tissue cells obtained from an adult lung).

According to a specific embodiment, the pulmonary tissue cells in suspension are administered to the subject at a dose range of about 40-1000 x 10⁶ cells per kilogram body weight of the subject (e.g. for administration of pulmonary tissue cells obtained from an adult lung).

According to a specific embodiment, the pulmonary tissue cells in suspension are administered to the subject at a dose range of about 1000-2000 x 10⁶ cells per kilogram body weight of the subject (e.g. for administration of pulmonary cells derived from an adult lung).

According to a specific embodiment, the pulmonary tissue cells in suspension are administered to the subject at a dose range of about 10-100 x 10⁶ cells per kilogram body weight of the subject (e.g. for administration of pulmonary tissue cells derived from a fetal lung).

According to a specific embodiment, the pulmonary tissue cells in suspension are administered to the subject at a dose range of about 40-100 x 10⁶ cells per kilogram body weight of the subject (e.g. for administration of pulmonary tissue cells derived from a fetal lung).

According to an embodiment of the present invention, the pulmonary tissue cells in suspension are administered to the subject at a dose of at least about 1 x 10⁶, 1.5 x 10⁶, 2 x 10⁶, 2.5 x 10⁶, 3 x 10⁶, 3.5 x 10⁶, 4 x 10⁶, 4.5 x 10⁶, 5 x 10⁶, 5.5 x 10⁶, 6 x 10⁶, 6.5 x 10⁶, 7 x 10⁶, 7.5 x 10⁶, 8 x 10⁶, 8.5 x 10⁶, 9 x 10⁶, 9.5 x 10⁶, 10 x 10⁶, 12.5 x 10⁶, 15 x 10⁶, 20 x 10⁶, 25 x 10⁶, 30 x 10⁶, 35 x 10⁶, 40 x 10⁶, 45 x 10⁶, 50 x 10⁶, 60 x 10⁶, 70 x 10⁶, 80 x 10⁶, 90 x 10⁶, 100 x 10⁶, 110 x 10⁶, 120 x 10⁶, 130 x 10⁶, 140 x 10⁶, 150 x 10⁶, 160 x 10⁶, 170 x 10⁶, 180 x 10⁶, 190 x 10⁶, 200 x 10⁶, 250 x 10⁶, 3 00 x 10⁶, 320 x 10⁶, 350 x 10⁶, 400 x 10⁶, 450 x 10⁶, 500 x 10⁶, 600 x 10⁶, 700 x 10⁶, 800 x 10⁶, 900 x 10⁶, 1000 x 10⁶, 1100 x 10⁶, 1200 x 10⁶, 1300 x 10⁶, 1400 x 10⁶, 1500 x 10⁶ or 2000 x 10⁶ cells per kilogram body weight of the subject.

According a specific embodiment, the pulmonary tissue cells in suspension are administered to the subject at a dose of at least about 40 x 10⁶ cells per Kg body weight of the subject (e.g. for administration of pulmonary tissue cells derived from an adult lung).

According a specific embodiment, the pulmonary tissue cells in suspension are administered to the subject at a dose of at least about 200 x 10⁶ cells per Kg body weight of the subject (e.g. for administration of pulmonary tissue cells derived from an adult lung).

According to one embodiment, the pulmonary tissue cells in suspension are administered to the subject at a dose of at least about 320 x 10⁶ per kilogram body weight of the subject (e.g. for administration of pulmonary tissue cells derived from an adult lung).

According to one embodiment, the pulmonary tissue cells in suspension are administered to the subject at a dose of at least about 500 x 10⁶ per kilogram body weight of the subject (e.g. for administration of pulmonary tissue cells derived from an adult lung).

According a specific embodiment, the pulmonary tissue cells in suspension are administered to the subject at a dose of at least about 1000 x 10⁶ cells per Kg body weight of the subject (e.g. for administration of pulmonary tissue cells derived from an adult lung).

According a specific embodiment, the pulmonary tissue cells in suspension are administered to the subject at a dose of at least about 2000 x 10⁶ cells per Kg body weight of the subject (e.g. for administration of pulmonary tissue cells derived from an adult lung).

According to one embodiment, the pulmonary tissue cells in suspension are administered to the subject at a dose of at least about 10 x 10⁶ per kilogram body weight of the subject (e.g. for administration of pulmonary tissue cells derived from a fetal lung).

According to one embodiment, the pulmonary tissue cells in suspension are administered to the subject at a dose of at least about 40 x 10⁶ per kilogram body weight of the subject (e.g. for administration of pulmonary tissue cells derived from a fetal lung).

According to one embodiment, the pulmonary tissue cells in suspension are administered to the subject at a dose of at least about 100 x 10⁶ per kilogram body weight of the subject (e.g. for administration of pulmonary tissue cells derived from a fetal lung).

According to a specific embodiment, the pulmonary tissue cells derived from a fetal lung (in suspension) are administered to the subject at a dose of about 10-100 x 10⁶ cells per kilogram body weight of the subject (e.g. about 40 x 10⁶ cells per kilogram body weight of the subject).

According to a specific embodiment, the pulmonary tissue cells obtained from an adult lung (in suspension) are administered to the subject at a dose of about 40-2000 x 10⁶ cells per kilogram body weight of the subject (e.g. about 320 x 10⁶ cells per kilogram body weight of the subject).

The pulmonary tissue cells in suspension comprising HPCs or supplemented with HPCs of some embodiments of the invention can be administered to an organism *per se,* or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the pulmonary tissue cells comprising HPCs or supplemented with HPCs accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intratracheal, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient (e.g. pulmonary tissue).

Pharmaceutical compositions may be manufactured by processes well known in the art, e.g., by means of conventional mixing, dissolving, granulating, dragee-making, levigating, emulsifying, encapsulating, entrapping or lyophilizing processes.

Pharmaceutical thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as crosslinked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients (i.e. pulmonary tissue cells in suspension comprising HPCs or supplemented with HPCs) effective to prevent, alleviate or ameliorate symptoms of a disorder (e.g., pulmonary disease or condition) or prolong the survival of the subject being treated.

Determination of a therapeutically effective amount is well within the capability of those skilled in the art, especially in light of the detailed disclosure provided herein.

For any preparation, the therapeutically effective amount or dose can be estimated initially from in vitro and cell culture assays. For example, a dose can be formulated in animal models to achieve a desired concentration or titer. Such information can be used to more accurately determine useful doses in humans.

An exemplary animal model which may be used to evaluate the therapeutically effective amount of pulmonary tissue cells in suspension comprising an effective amount of HPCs or supplemented with HPCs comprises the murine animal model (e.g. mice), in which lung injury is induced by e.g. intraperitoneal injection of naphthalene (e.g. more than 99 % pure) with or without further irradiation (e.g. 40-48 hours after naphthalene administration), as described in detail in the Examples section which follows.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide ample levels of the active ingredient which are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation formulated in a compatible pharmaceutical carrier may also be prepared, placed in an appropriate container, and labeled for treatment of an indicated condition, as is further detailed above.

To further avoid residual immune reaction which may still be present when administering pulmonary tissue cells, several approaches have been developed to reduce the likelihood of rejection. These include encapsulating the non-syngeneic cells in immunoisolating, semipermeable membranes before transplantation. Alternatively, cells may be uses which do not express xenogenic surface antigens, such as those developed in transgenic animals (e.g. pigs).

Encapsulation techniques are generally classified as microencapsulation, involving small spherical vehicles, and macroencapsulation, involving larger flat-sheet and hollow-fiber membranes (Uludag, H. et al. (2000). Technology of mammalian cell encapsulation. Adv Drug Deliv Rev 42, 29-64).

Methods of preparing microcapsules are known in the art and include for example those disclosed in: Lu, M. Z. et al. (2000). Cell encapsulation with alginate and alpha-phenoxycinnamylidene-acetylated poly(allylamine). Biotechnol Bioeng 70, 479-483; Chang, T. M. and Prakash, S. (2001) Procedures for microencapsulation of enzymes, cells and genetically engineered microorganisms. Mol Biotechnol 17, 249-260; and Lu, M. Z., et al. (2000). A novel cell encapsulation method using photosensitive poly(allylamine alpha-cyanocinnamylideneacetate). J Microencapsul 17, 245-521.

For example, microcapsules are prepared using modified collagen in a complex with a ter-polymer shell of 2-hydroxyethyl methylacrylate (HEMA), methacrylic acid (MAA), and methyl methacrylate (MMA), resulting in a capsule thickness of 2-5 µm. Such microcapsules can be further encapsulated with an additional 2-5 µm of ter-polymer shells in order to impart a negatively charged smooth surface and to minimize plasma protein absorption (Chia, S. M. et al. (2002). Multi-layered microcapsules for cell encapsulation. Biomaterials 23, 849-856).

Other microcapsules are based on alginate, a marine polysaccharide (Sambanis, A. (2003). Encapsulated islets in diabetes treatment. Diabetes Thechnol Ther 5, 665-668), or its derivatives. For example, microcapsules can be prepared by the polyelectrolyte complexation between the polyanions sodium alginate and sodium cellulose sulphate and the polycation poly (methylene-co-guanidine) hydrochloride in the presence of calcium chloride.

It will be appreciated that cell encapsulation is improved when smaller capsules are used. Thus, for instance, the quality control, mechanical stability, diffusion properties, and *in vitro* activities of encapsulated cells improved when the capsule size was reduced from 1 mm to 400 µm (Canaple, L. et al. (2002). Improving cell encapsulation through size control. J Biomater Sci Polym Ed 13, 783-96). Moreover, nanoporous biocapsules with well-controlled pore size as small as 7 nm, tailored surface chemistries, and precise microarchitectures were found to successfully immunoisolate microenvironments for cells (See: Williams, D. (1999). Small is beautiful: microparticle and nanoparticle technology in medical devices. Med Device Technol 10, 6-9; and Desai, T. A. (2002). Microfabrication technology for pancreatic cell encapsulation. Expert Opin Biol Ther 2, 633-646).

According to one embodiment, transplantation of the pulmonary tissue cells results in regenerating of structural/functional pulmonary tissue.

According to one embodiment, transplantation of the pulmonary tissue cells results in generation of a chimeric lung (i.e. a lung comprising cells from genetically distinct origins).

It will be appreciated that the cells within the pulmonary tissue cells in suspension are capable of regenerating a structural/functional pulmonary tissue, including generation of a chimeric lung. The chimeric lung comprises alveolar, bronchial and/or bronchiolar structures, and/or vascular structures. Furthermore, the structural/functional pulmonary tissue comprises an ability to synthesize surfactant [e.g. clara cell secretory protein (CCSP), aquqporin-5 (AQP-5) and surfactant protein C (sp-C)], detectable by specific cell staining, and/or an ability to transport ions (e.g. as indicated by staining for CFTR-cystic fibrosis transmembrane regulator). The cells within the pulmonary tissue cells in suspension are further capable of regenerating an epithelial, mesenchymal and/or endothelial tissue (e.g. epithelial, mesenchymal and/or endothelial tissue, as indicated by the formation of a complete chimeric lung tissue comprising all of these components).

Following transplantation of the pulmonary tissue cells into the subject according to the present teachings, it is advisable, according to standard medical practice, to monitor the growth functionality and immunocompatability of the transplanted cells according to any one of various standard art techniques. For example, the functionality of regenerated pulmonary tissues may be monitored following transplantation by standard pulmonary function tests, e.g. by analysis of functional properties of the developing implants, as indicated by the ability to synthesize surfactant, detectable by staining for surfactant protein C (sp-C) and the ability to transport ions, as indicated by staining for CFTR-cystic fibrosis transmembrane regulator.

In order to facilitate engraftment of the pulmonary tissue cells, and in order to reduce, by at least about 30 %, 40 %, 50 %, 60 %, 70 %, 80 %, 90 % or 95 %, or preferably avoid graft rejection and/or graft versus host disease (GVHD), the method may further advantageously comprise conditioning the subject prior to administration of the pulmonary tissue cells.

As used herein, the term "conditioning" refers to the preparative treatment of a subject prior to transplantation.

According to one embodiment, to increase the rate of a successful transplantation (e.g. the formation of chimerism) the subject is treated by a conditioning capable of vacating cell niches in the pulmonary tissue or organ.

Thus, conditioning the subject is effected by administering to a subject a therapeutically effective amount of an agent capable of inducing damage to the pulmonary tissue wherein the damage results in proliferation of resident stem cells in the pulmonary tissue.

The phrase "damage to the pulmonary tissue" refers to a localized injury to a pulmonary organ/tissue or a part thereof.

The term "proliferation of resident stem cells" refers to the induction of cell division of endogenous stem cells residing within the pulmonary tissue once subjected to the agent.

Various conditioning agents may be used in accordance with the present invention as long as the agent induces damage to at least a part of the pulmonary tissue which results in proliferation of resident stem cells within the pulmonary tissue. Thus, for example, the agent may comprise a chemical, an antibiotic, a therapeutic drug, a toxin or an herb or an extract thereof.

The conditioning protocol may be adjusted taking into consideration the age and condition (e.g. disease, disease stage) of the subject, such a determination is well within the capacity of those of skill in the art, especially in view of the disclosure provided herein.

Without being bound to theory, a therapeutically effective amount of conditioning is an amount of the conditioning agent sufficient for inducing localized pulmonary tissue damage and proliferation of resident stem cells, but not being toxic to other organs of the subject being treated (e.g. liver, kidneys, heart, etc.).

Determination of the therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired concentration or titer. Such information can be used to more accurately determine useful doses in humans.

Exemplary agents causing pulmonary cell toxicity, include but are not limited to, chemotherapeutic agents, immunosuppressive agents, amiodarone, beta blockers, ACE inhibitors, nitrofurantoin, procainamide, quinidine, tocainide, minoxidil, amiodarone, methotrexate, taxanes (e.g. paclitaxel and docetaxel), gemcitabine, bleomycin, mitomycin C, busulfan, cyclophosphamide, chlorambucil, nitrosourea (e.g., carmustine) and Sirolimus.

Additional agents causing pulmonary cell toxicity are listed in Table 2, below [incorporated from Collard, www(dot)merckmanuals(dot)com/professional/pulmonary-disorders/interstitial-lung-diseases/drug-induced-pulmonary-disease].

**Table 2: Substances with toxic pulmonary effects**

| ***Condition*** | ***Drug or Agent*** |
|---|---|
| Asthma | Aspirin, β-blockers (e.g., timolol), cocaine, dipyridamole, IV hydrocortisone, IL-2, methylphenidate, nitrofurantoin, protamine, sulfasalazine, vinca alkaloids (with mitomycin-C) |
| Organizing pneumonia | Amiodarone, bleomycin, cocaine, cyclophosphamide, methotrexate, minocycline, mitomycin-C, penicillamine, sulfasalazine, tetracycline |
| Hypersensitivity pneumonitis | Azathioprine plus 6-mercaptopurine, busulfan, fluoxetine, radiation |
| Interstitial pneumonia or fibrosis | Amphotericin B, bleomycin, busulfan, carbamazepine, chlorambucil, cocaine, cyclophosphamide, diphenylhydantoin, flecainide, heroin, melphalan, methadone, methotrexate, methylphenidate, methysergide, mineral oil (via chronic microaspiration), nitrofurantoin, nitrosoureas, procarbazine, silicone (s.c. injection), tocainide, vinca alkaloids (with mitomycin-C) |
| Noncardiac pulmonary edema | β-Adrenergic agonists (e.g., ritodrine, terbutaline), chlordiazepoxide, cocaine, cytarabine, ethiodized oil (IV, and via chronic microaspiration), gemcitabine, heroin, hydrochlorothiazide, methadone, mitomycin-C, phenothiazines, protamine, sulfasalazine, tocolytic agents, tricyclic antidepressants, tumor necrosis factor, vinca alkaloids (with mitomycin-C) |
| Parenchymal hemorrhage | Anticoagulants, azathioprine plus 6-mercaptopurine, cocaine, mineral oil (via chronic microaspiration), nitrofurantoin, radiation |
| Pleural effusion | Amiodarone, anticoagulants, bleomycin, bromocriptine, busulfan, granulocyte-macrophage colony-stimulating factor, IL-2, methotrexate, methysergide, mitomycin-C, nitrofurantoin, para-aminosalicylic acid, procarbazine, radiation, tocolytic agents |
| Pulmonary infiltrate with eosinophilia | Amiodarone, amphotericin B, bleomycin, carbamazepine, diphenylhydantoin, ethambutol, etoposide, granulocyte-macrophage colony-stimulating factor, isoniazid, methotrexate, minocycline, mitomycin-C, nitrofurantoin, para-aminosalicylic acid, procarbazine, radiation, sulfasalazine, sulfonamides, tetracycline, trazodone |
| Pulmonary vascular disease | Appetite suppressants (e.g., dexfenfluramine, fenfluramine, phentermine), busulfan, cocaine, heroin, methadone, methylphenidate, nitrosoureas, radiation |

As illustrated in the Examples section which follows, conditioning a subject using naphthalene induces site-specific ablation (e.g. of Clara cells in respiratory bronchioles and in broncho-alveolar junctions) and thus facilitate engraftment of the pulmonary tissue cells in suspension. To further effectively eliminate residential lung stem cells (which may proliferate rapidly after naphthalene treatment), subject were further subjected to sublethal TBI (e.g. 6 Gy) prior to administration of the pulmonary tissue cells in suspension (see Example 1 and Figure 3 of the Examples section which follows).

Thus, according to one embodiment of the present invention, the conditioning protocol comprises Naphthalene treatment.

According to one embodiment, Naphthalene treatment is administered to the subject 1-10 days (e.g. 7, 6, 5, 4, 3, 2 days, e.g. 3 days) prior to administration of the pulmonary tissue cells in suspension.

Assessing pulmonary tissue damage can be carried out using any method known in the art, e.g. by pulmonary function tests, chest X-ray, by chest CT, or by PET scan. Determination of pulmonary damage is well within the capability of those skilled in the art, especially in light of the detailed disclosure provided herein.

As described above, pulmonary tissue damage results in proliferation of resident stem cells within the tissue.

Assessing proliferation of resident stem cells (e.g. endogenous stem cells within a pulmonary tissue) can be carried out using any method know to one of skill in the art, such as for example, by *in-vivo* imaging of cellular proliferation e.g. using a Positron emission tomography (PET) with a PET tracer e.g. 18F labeled 2-fluoro-2-deoxy-D-glucose (18FDG) or [18F] 3'-deoxy-3-fluorothymidine ((18)FLT) as taught by Francis et al, Gut. (2003) 52(11):1602-6 and by Fuchs et al., J Nucl Med. (2013) 54(1):151-8.

Thus, according to one embodiment of the invention, following administration of the agent capable of inducing damage to the tissue of interest, the subject is subjected to a second conditioning agent, i.e. an agent which ablates the resident stem cells in the tissue. As will be apparent to those of ordinary skill in the art of cell biology, sensitivity to radiation is achieved only in a proliferative stage.

According to another embodiment, an agent which ablates the resident stem cells in the tissue (as discussed below) can be administered to the subject without prior conditioning with an agent which induces damage to the tissue (e.g. naphthalene).

According to one embodiment, the agent which ablates the resident stem cells comprises a sublethal, lethal or supralethal conditioning protocol.

According to one embodiment, the conditioning protocol comprises reduced intensity conditioning (RIC).

According to an embodiment, the reduced intensity conditioning is effected for up to 2 weeks (e.g. 1-14, 1-10 or 1-7 days) prior to transplantation of the pulmonary tissue cells.

According to one embodiment, the conditioning protocol comprises a total body irradiation (TBI), total lymphoid irradiation (TLI, i.e. exposure of all lymph nodes, the thymus, and spleen), partial body irradiation, T cell debulking (TCD), a chemotherapeutic agent and/or an antibody immunotherapy.

Thus, according to one embodiment, the TBI comprises a single or fractionated irradiation dose within the range of 0.5-1 Gy, 0.5-1.5 Gy, 0.5-2.5 Gy, 0.5-5 Gy, 0.5-7.5 Gy, 0.5-10 Gy, 0.5-15 Gy, 0.5-20 Gy, 1-1.5 Gy, 1-2 Gy, 1-2.5 Gy, 1-3 Gy, 1-3.5 Gy, 1-4 Gy, 1-4.5 Gy, 1-1.5 Gy, 1-7.5 Gy, 1-10, Gy, 1-15, Gy, 1-12 Gy, 2-3 Gy, 2-4 Gy, 2-5 Gy, 2-6 Gy, 2-7 Gy, 2-8 Gy, 2-9 Gy, 2-10 Gy, 2-15 Gy, 2-20 Gy, 3-4 Gy, 3-5 Gy, 3-6 Gy, 3-7 Gy, 3-8 Gy, 3-9 Gy, 3-10 Gy, 3-15 Gy, 3-20 Gy, 4-5 Gy, 4-6 Gy, 4-7 Gy, 4-8 Gy, 4-9 Gy, 4-10 Gy, 4-15 Gy, 4-20 Gy, 5-6 Gy, 5-7 Gy, 5-8 Gy, 5-9 Gy, 5-10 Gy, 5-15 Gy, 5-20 Gy, 6-7 Gy, 6-8 Gy, 6-9 Gy, 6-10 Gy, 6-20 Gy, 7-8 Gy, 7-9 Gy, 7-10 Gy, 7-20 Gy, 8-9, Gy, 8-10 Gy, 10-12 Gy, 10-15 Gy or 10-20 Gy.

According to a specific embodiment, the TBI comprises a single or fractionated irradiation dose within the range of 1-20 Gy.

According to a specific embodiment, the TBI comprises a single or fractionated irradiation dose within the range of 1-10 Gy.

According to an embodiment, TBI treatment is administered to the subject 1-10 days (e.g. 1-3 days) prior to transplantation. According to one embodiment, the subject is conditioned once with TBI 1 or 2 days prior to transplantation.

According to a specific embodiment, the TLI comprises an irradiation dose within the range of 0.5-1 Gy, 0.5-1.5 Gy, 0.5-2.5 Gy, 0.5-5 Gy, 0.5-7.5 Gy, 0.5-10 Gy, 0.5-15 Gy, 0.5-20 Gy, 1-1.5 Gy, 1-2 Gy, 1-2.5 Gy, 1-3 Gy, 1-3.5 Gy, 1-4 Gy, 1-4.5 Gy, 1-1.5 Gy, 1-7.5 Gy, 1-10 Gy, 2-3 Gy, 2-4 Gy, 2-5 Gy, 2-6 Gy, 2-7 Gy, 2-8 Gy, 2-9 Gy, 2-10 Gy, 3-4 Gy, 3-5 Gy, 3-6 Gy, 3-7 Gy, 3-8 Gy, 3-9 Gy, 3-10 Gy, 4-5 Gy, 4-6 Gy, 4-7 Gy, 4-8 Gy, 4-9 Gy, 4-10 Gy, 5-6 Gy, 5-7 Gy, 5-8 Gy, 5-9 Gy, 5-10 Gy, 6-7 Gy, 6-8 Gy, 6-9 Gy, 6-10 Gy, 7-8 Gy, 7-9 Gy, 7-10 Gy, 8-9 Gy, 8-10 Gy, 10-12 Gy, 10-15 Gy, 10-20 Gy, 10-30 Gy, 10-40 Gy, 10-50 Gy, 0.5-20 Gy, 0.5-30 Gy, 0.5-40 Gy or 0.5-50 Gy.

According to a specific embodiment, the TLI comprises a single or fractionated irradiation dose within the range of 1-20 Gy.

According to a specific embodiment, the TLI comprises a single or fractionated irradiation dose within the range of 1 -10 Gy.

According to an embodiment, TLI treatment is administered to the subject 1-10 days (e.g. 1-3 days) prior to transplantation. According to one embodiment, the subject is conditioned once with TLI 1 or 2 days prior to transplantation.

As described in detail in the Examples section which follows, the subject may be treated by *in-vivo* T cell debulking e.g. by anti-CD4 antibody, anti-CD8 antibody, anti-CD3 (OKT3) antibody, anti-CD52 antibody (e.g. CAMPATH) and/or anti-thymocyte globulin (ATG) antibody (e.g. 10, 9, 8, 7, 6 or 5 days prior to transplantation at a therapeutic effective dose of about 100-500 µg, e.g. 300 µg each).

According to one embodiment, the conditioning comprises a chemotherapeutic agent. Exemplary chemotherapeutic agents include, but are not limited to, Busulfan, Myleran, Busulfex, Fludarabine, Melphalan, Dimethyl mileran and Thiotepa and cyclophosphamide. The chemotherapeutic agent/s may be administered to the subject in a single dose or in several doses e.g. 2, 3, 4, 5, 6, 7, 8, 9, 10 or more doses (e.g. daily doses) prior to transplantation. According to one embodiment, the subject is administered a chemotherapeutic agent (e.g. Fludarabine e.g. at a dose of about 30 mg/m2/day) for 3-7 consecutive days, e.g. 5 consecutive days, prior to transplantation (e.g. on days -7 to -3).

According to one embodiment, the conditioning comprises an antibody immunotherapy. Exemplary antibodies include, but are not limited to, an anti-CD52 antibody (e.g. Alemtuzumab sold under the brand names of e.g. Campath, MabCampath, Campath-1H and Lemtrada) and an anti-thymocyte globulin (ATG) agent [e.g. Thymoglobulin (rabbit ATG, rATG, available from Genzyme) and Atgam (equine ATG, eATG, available from Pfizer)]. Additional antibody immunotherapy may comprise anti-CD3 (OKT3), anti-CD4 or anti-CD8 agents. According to one embodiment, the antibody is administered to the subject in a single dose or in several doses e.g. 2, 3, 4, 5, 6, 7, 8, 9, 10 or more doses (e.g. daily doses) prior to transplantation (e.g. 4-8 days, e.g. 6 days, prior to transplantation).

According to one embodiment, the conditioning comprises co-stimulatory blockade. Thus, for example, the conditioning may comprise transiently administering to the subject at least one T-cell co-stimulation inhibitor and at least one CD40 ligand inhibitor, and more preferably may further comprise administering to the subject an inhibitor of T-cell proliferation.

According to one embodiment, the T-cell co-stimulation inhibitor is CTLA4-Ig, the CD40 ligand inhibitor is anti-CD40 ligand antibody, and the inhibitor of T-cell proliferation is rapamycin. Alternately, the T-cell co-stimulation inhibitor may be an anti-CD40 antibody. Alternately, the T-cell co-stimulation inhibitor may be an antibody specific for B7-1, B7-2, CD28, anti-LFA-1 and/or anti-LFA3.

According to a specific embodiment, the conditioning comprises Naphthalene treatment (e.g. 10, 9, 8, 7, 6, 5, 4, 3 or 2 days, e.g. 3 days, prior to transplantation) and TBI treatment (e.g. 9, 8, 7, 6, 5, 4, 3, 2 or 1 days, e.g. 1 day, prior to transplantation, at a dose of e.g. 1-20 Gy, e.g. 6 Gy).

According to another specific embodiment, the conditioning comprises T cell debulking treatment (e.g. 10, 9, 8, 7, 6, 5, 4, 3 or 2 days, e.g. 6 days, prior to transplantation, e.g. with anti-CD8 and/or anti-CD4 antibodies), Naphthalene treatment (e.g. 10, 9, 8, 7, 6, 5, 4, 3 or 2 days, e.g. 3 days, prior to transplantation) and TBI treatment (e.g. 9, 8, 7, 6, 5, 4, 3, 2 or 1 days, e.g. 1 day, prior to transplantation, at a dose of e.g. 1-20 Gy, e.g. 6 Gy).

According to another specific embodiment, the conditioning comprises only TBI treatment (e.g. 9, 8, 7, 6, 5, 4, 3, 2 or 1 days, e.g. 1 day, prior to transplantation, at a dose of e.g. 1-20 Gy, e.g. 6 Gy).

In order to avoid graft rejection of the pulmonary tissue cells, the subject may be administered with a post-transplant immunosuppressive regimen.

According to one embodiment, the subjected is treated with an immunosuppressive regimen for up to two weeks following administration of the pulmonary tissue cells.

According to one embodiment, the subject is treated with an immunosuppressive regimen for up to 5 days, 6 days, 7 days, 8 days, 9 days, 10 days, 11 days, 12 days, 13 days or 14 days following administration of the pulmonary tissue cells.

Examples of suitable types of immunosuppressive regimens include administration of immunosuppressive drugs (also termed immunosuppressive agents) and/or immunosuppressive irradiation.

Ample guidance for selecting and administering suitable immunosuppressive regimens for transplantation is provided in the literature of the art (for example, refer to: Kirkpatrick CH. and Rowlands DT Jr., 1992. JAMA. 268, 2952; Higgins RM. et al., 1996. Lancet 348, 1208; Suthanthiran M. and Strom TB., 1996. New Engl. J. Med. 331, 365; Midthun DE. et al. 1997. Mayo Clin Proc. 72, 175; Morrison VA. et al. 1994. Am J Med. 97, 14; Hanto DW., 1995. Annu Rev Med. 46, 381; Senderowicz AM. et al., 1997. Ann Intern Med. 126, 882; Vincenti F. et al., 1998. New Engl. J. Med. 338, 161; Dantal J. et al. 1998. Lancet 351, 623).

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE), etanercept, TNF.alpha. blockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors, tramadol, rapamycin (sirolimus) and rapamycin analogs (such as CCI-779, RAD001, AP23573). These agents may be administered individually or in combination.

According to one embodiment, the immunosuppressive agent is cyclophosphamide.

As used herein, the term "cyclophosphamide" refers to the nitrogen mustard alkylating agent which specifically adds an alkyl group (CnH2n+1) to DNA (also known as cytophosphane). In a specific embodiment, the cyclophosphamide refers to the molecular formula C₇H₁₅C₁₂N₂O₂P•H₂O and the chemical name 2-[bis(2-chloroethyl)amino]tetrahydro-2H-1,3,2-oxazaphosphorine 2-oxide monohydrate. Cyclophosphamide is commercially available from e.g. Zydus (German Remedies), Roxane Laboratories Inc-Boehringer Ingelheim, Bristol-Myers Squibb Co - Mead Johnson and Co, and Pfizer - Pharmacia & Upjohn, under the brand names of Endoxan, Cytoxan, Neosar, Procytox and Revimmune.

A therapeutically effective amount of cyclophosphamide is typically administered to the subject following transplantation of the pulmonary tissue cells.

According to one embodiment, the present invention further contemplates administration of cyclophosphamide prior to transplantation (e.g. on days 4, 3 or 2 prior to transplantation, i.e. T-4, -3 or -2) in addition to the administration following transplantation as described herein.

Of note, the date of transplantation (of the pulmonary tissue cells) is considered T=zero.

Without being bound to theory, a therapeutically effective amount is an amount of cyclophosphamide efficient for killing activated donor or host alloreactive T cells without being toxic to the subject.

For example, in case of transplantation of pulmonary tissue cells, the therapeutic effective amount of cyclophosphamide comprises about 1-25 mg, 1-50 mg, 1-75 mg, 1-100 mg, 1-250 mg, 1-500 mg, 1-750 mg, 1-1000 mg, 5-50 mg, 5-75 mg, 5-100 mg, 5-250 mg, 5-500 mg, 5-750 mg, 5-1000 mg, 10-50 mg, 10-75 mg, 10-100 mg, 10-250 mg, 10-500 mg, 10-750 mg, 10-1000 mg, 25-50 mg, 25-75 mg, 25-100 mg, 25-125 mg, 25-200 mg, 25-300 mg, 25-400 mg, 25-500 mg, 25-750 mg, 25-1000 mg, 50-75 mg, 50-100 mg, 50-125 mg, 50-150 mg, 50-175 mg, 50-200 mg, 50-250 mg, 50-500 mg, 50-1000 mg, 75-100 mg, 75-125 mg, 75-150 mg, 75-250 mg, 75-500 mg, 75-1000 mg, 100-125 mg, 100-150 mg, 100-200 mg, 100-300 mg, 100-400 mg, 100-500 mg, 100-1000 mg, 125-150 mg, 125-250 mg, 125-500 mg, 125-1000 mg, 150-200 mg, 150-300 mg, 150-500 mg, 150-1000 mg, 200-300 mg, 200-400 mg, 200-500 mg, 200-750 mg, 200-1000 mg, 250-500 mg, 250-750 mg, 250-1000 mg per kilogram body weight of the subject.

According to a specific embodiment, the therapeutic effective amount of cyclophosphamide is about 25-200 mg per kilogram body weight of the subject.

According to a specific embodiment, the therapeutic effective amount of cyclophosphamide is about 50-150 mg per kilogram body weight of the subject.

According to a specific embodiment, the therapeutic effective amount of cyclophosphamide is about 100 mg per kilogram body weight of the subject.

As illustrated in the Examples section which follows, the present inventors have shown that administration of two doses of cyclophosphamide post-transplant (on days 3 and 4 post-transplant) allows for a durable engraftment and tolerance of 'mega dose' T cell depleted pulmonary tissue cells.

According to one embodiment, cyclophosphamide is administered in a single dose.

According to one embodiment, cyclophosphamide is administered in multiple doses, e.g. in 2, 3, 4, 5 doses or more.

According to a specific embodiment, cyclophosphamide is administered in two doses.

According to one embodiment, cyclophosphamide is administered daily such as once a day or twice a day.

The dose of each cyclophosphamide administration may comprise about 5 mg, 7.5 mg, 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg or 500 mg per kilogram body weight of the subject.

According to a specific embodiment, the dose of cyclophosphamide is 50 mg per kilogram body weight of the subject.

According to one embodiment, cyclophosphamide is administered post transplantation. Thus, for example, cyclophosphamide may be administered to the subject 1, 2, 3, 4, 5, 6, 7, 8, 9, 10 days or more post-transplant (i.e., T+1, +2, +3, +4, +5, +6, +7, +8, +9, +10). According to a specific embodiment, cyclophosphamide is administered to the subject in two doses, e.g. on days 3 and 4 days post-transplant.

According to an embodiment, cyclophosphamide is administered prior to transplantation and post transplantation. Thus, for example, cyclophosphamide may be administered to the subject 3 days prior to transplantation (T-3) and then post transplantation (e.g. on days T+3, +4, etc.).

The number of administrations and the therapeutically effective amount of cyclophosphamide may be adjusted as needed taking into account the type of transplantation and the subject's response to the regimen. Determination of the number of administrations and the therapeutically effective amount is well within the capability of those skilled in the art, especially in light of the detailed disclosure provided herein.

As used herein the term "about" refers to ± 10 %.

The terms "comprises", "comprising", "includes", "including", "having" and their conjugates mean "including but not limited to".

The term "consisting of' means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions, illustrate the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### GENERAL MATERIALS AND EXPERIMENTAL PROCEDURES

### Animals

Animals were maintained under conditions approved by the Institutional Animal Care and Use Committee at the Weizmann Institute. All of the procedures were monitored by the veterinarian of the Veterinary Resources Unit of the Weizmann Institute, and approved by the Institutional Animal Care and Use Committee (IACUC).

Mice strains used included: C57BL/6J (CD45.2 and CD45.1), beta-actin GFP on C57BL/6J background, C3H/HeJ (Weizmann Institute Animal Breeding Center, Rehovot, Israel), Rag1^{-/-} mice on a C57BL/6J background (Weizmann breeding center), and tdTomato (Gt(ROSA)26Sortm4) (ACTB-tdTomato,-EGFP) Luo/J mice (Jackson Labs, Bar Harbor, USA).

All mice were used at 6-18 weeks of age. Mice were kept in small cages (up to five animals in each cage) and fed sterile food and acid water. Randomization: animals of the same age, sex and genetic background were randomly assigned to treatment groups. Preestablished exclusion criteria were based on IACUC guidelines, and included systemic disease, toxicity, respiratory distress, refusal to eat and drink, and substantial (more than 15 %) weight loss. During the study period, more than 90 % of the mice appeared to be in good health and were included in the appropriate analysis. In all experiments, the animals were randomly assigned to the treatment groups.

For the experiment shown in Figures 16A-D, C57BL/6J and C57BL/6J E16 female mice were used. For Figures 17A-G, each group of five female C57BL/6J mice was used for plating 12 well plates. For Figures 9A-E and 10A-B, in experiments studying dose response, conditioned C57BL/6J females, aged 8 weeks, were used as hosts, with four to five mice in each group, for two experiments. C57BL/6J - GFP female mice, aged 8-18 weeks, and E15-16 - GFP positive female mice were used as lung donors. For Figures 9A-E and 10A-B, seven conditioned C57BL/6J females were used as hosts, and C57BL/6J -GFP⁺ female mice as lung donors. For the experiment shown in Figures 11A-G, conditioned Ragl^{-/-}-C57BL/6J female mice (aged 8-12 weeks) were transplanted with a 1:1 mixture of 6 × 10⁶ GFP⁺ and tdTomato-positive adult lung cells.

For Figure 15 (lung function measurements), C57BL/6J mice were transplanted in two experiments with 4 × 10⁶ or 6 × 10⁶ adult lung cells from C57BL/6J -GFP⁺ donor mice. C57BL/6J with and without lung damage were used as controls in both experiments.

### Mixed chimerism assay

Mice irradiated with 10 Gy were transplanted by syngeneic bone marrow CD45.1 along with embryonic lung derived from beta-actin GFP (CD45.2) single cell suspension at a 1:1 relation - total of 10⁶ cells.

### Induction of lung injury

For the lung injury studies, naphthalene (more than 99 % pure; Sigma-Aldrich) was dissolved in corn oil, and administered (at a dose of 200 mg per kg body weight) to C57BL/6 mice, by intraperitoneal (IP) injection, 3 days before transplantation of embryonic lung cells, as previously described [Stripp, B.R., et al., American Journal of Physiology- Lung Cellular and Molecular Physiology (1995) 269(6): p. 791].

For "double lung" injury, naphthalene-treated animals were further irradiated in an X-ray irradiator (Xrad-320), 40-48 hours after naphthalene administration, for a total dose of 6 Gy TBI, and transplanted 4 to 24 hours later.

### T cell debulking (TCD) treatment

Recipient mice were injected IP with TCD 300 µg/mouse of anti-CD4 (clone GK1.5) and anti-CD8 (clone YTS169.4) antibodies 6 days before the transplantation of embryonic lung cells.

### Cyclophosphamide (Endoxan) treatment

Recipient mice were injected IP with CY 100 mg/kg at days 3-4 post transplantation of embryonic lung cells (days +3 and +4).

### Mouse fetal lung single cell suspension: cell preparation and transplantation procedures

Cell suspensions were obtained from enzyme-digested mouse adult and embryo lungs, as previously described [Liang S.X. et al., Physiological genomics. (2005) 23(2): 172]. Briefly, lung digestion was performed by finely mincing tissue with a razor blade in the presence of 1 mg/ml collagenase (Roche Diagnostics, Indianapolis, IN) in PBS Ca⁺Mg⁺. After incubation for 30 minutes at 37 °C, an 18-G needle was used to triturate the chunks of the tissue. Following another incubation for 30 minutes at 37 °C, an additional trituration with a 21-G needle was performed. Nonspecific debris were removed by sequential filtration through 100-µm filters. The cells were then washed with 1 × PBS (Ca and Mg free) with 2 % FCS. To prevent cell clumping before injection, 50 units heparin/ml were added to the single cell suspension before i.v. injection, and the suspension was filtered again through 40-µm filters.

The isolated cell suspension was depleted of T cells using magnetic beads for CD4 and CD8.

Following conditioning with both Naphthalene (on day -3) and TBI (day -1), C57BL/6J mice were transplanted with 1 x 10⁶ to 8 x 10⁶ GFP-positive adult or E15-16 embryo lung cells injected into the tail vein 4-24 hours following irradiation. In experiments using GFP and tdTomato labeling, *Rag1*^{*-*/*-*} -C57BL/6J donor mice were transplanted with a 1:1 mixture of 6 x 10⁶ GFP- and tdTomato-positive lung cells injected into the tail vein, 4-24 hours following irradiation.

### E16 lung expanded cells preparation and injection

GFP positive C57BL E16 lung cells were harvested and seeded on tissue culture plates with condition medium [irradiated mouse embryonic feeders (iMEF)] together with epithelial growth factor (1 µM) and Rock inhibitor (Y-27632, 5 µM). Medium was changed every 2 days and Rock inhibitor was added freshly every time. After 4 days cells were passed by splitting them to 3 plates. After 3 additional days on culture the cells were used for transplantation. A single cell suspension comprising 2 x 10⁶ expanded cells were injected i.v. into C57BL/6 recipient mice following conditioning with NA and 6 GY TBI.

### Fresh adult lung cells preparation and injection

Adult lung cells were harvested from a GFP positive C57BL/6 mice (Jackson Labs, Bar Harbor, USA). All mice were 6-12 weeks of age. A single cell suspension comprising 8 x 10⁶ adult lung cells were injected i.v. into C57BL/6 recipient mice following conditioning with NA and 6 GY TBI.

### Assessment of GFP⁺ foci in chimeric lungs by morphometry

Lungs were fixed with a 4 % PFA solution introduced through the trachea under a constant pressure of 20 cm H₂O. Then the lungs were immersed in fixative overnight at 4 °C. Lungs were processed after PFA treatment and fixed in 30 % sucrose and frozen in Optimal Cutting Temperature (OCT) compound (Sakura Finetek USA, Inc.Tissue-Tek). Serial step sections, 12 µm in thickness, were taken along the longitudinal axis of the lobe. The fixed distance between the sections was calculated so as to allow systematic sampling of at least 20 sections across the whole lung. Lung slices were analyzed by fluorescence microscopy. The actual number of GFP⁺ foci (a group of more than 5 distinct GFP⁺ cells was defined as a single patch) was counted per slice using Image Pro software. The area of each slice was estimated by Image Pro software, and the average area of slices and average frequency of GFP⁺ patches in all the slices were assessed (patch(P) / Area(A) (mm2)), assuming that the frequency per area in a large number of slices reflects distribution per volume.

### Flow cytometry (FACS)

Peripheral blood cells, bone marrow (BM), fetal liver and fetal lung of mice were analyzed by flow cytometry.

Single cell suspensions from mouse adult and fetal lungs were prepared by enzymatic digestion and analyzed by polychromatic flow cytometry. Samples were stained with conjugated antibodies or matching isotype controls according to the manufacturer's instructions. Antibodies were purchased from e-Bioscience, BD and Biolegend. The complete list of antibodies used in the study is provided (SI 4). Data were acquired on an LSRII (BD Biosciences) or BD FACSCanto II flow cytometer, and analyzed using BD FACSDiva 6 or FlowJo software (version 7.6.5, or version vX.0.7 Tree Star Inc).

### Immunohistochemistry (IHC)

Animals were sacrificed at different time points following transplantation; the lungs were inflated to full capacity with 4 % PFA solution and maintained for 24 hours, then cryopreserved in 30 % sucrose, and snap frozen in isopentane precooled by liquid air. Frozen samples were cut into 12-µm sections and stained. The list of antibodies and dilutions used in this study is provided in Table 3, hereinbelow. All secondary antibodies were purchased from Jackson Immunoresearch Laboratories or Abcam.

The stained samples were evaluated using an upright Olympus BX51 fluorescent microscope with ×10, ×40 air and ×100 oil objectives, and Olympus digital camera (DP70), or by Nikon Eclipse T*i* inverted spinning disc confocal microscope with ×10, ×20 air objectives and ×40, ×60 and ×100 oil objectives for high resolution. Fluorescence microscopy images were acquired by DP Controller and DP Manager software (Olympus). Confocal microscopy images were acquired using Andor iQ software, and analyzed and reconstructed in three dimensions (as indicated) with Imaris software (Bitplane AG, Switzerland, www.bitplane.com). In some cases, images were processed (intensity and contrast adjusted, overlaid) in Adobe Photoshop.

**Table 3: A list of the antibodies used in the study**

| **Primary antibodies** | **Application** | **Catalog number** | **Dilution** |
|---|---|---|---|
| Rabbit anti- CK5 (Abcam) | IHC | Ab53121 Ab52635 | 1:100 |
| Goat anti-mouse nestin (Santa-Cruz) | IHC | Sc-21249 | 1:100 |
| Chicken anti- GFP (Abcam) | IHC | Ab13970 | 1:500 |
| Rabbit anti- surfactant protein C (Santa-Cruz | IHC | Sc-13979 (FL-197) | 1:100 |
| Goat anti-Aquaporin-5 (Santa-Cruz) | IHC | Sc-9890 | 1:100 |
| Rabbit anti-Aquaporin5 (Millipore) | IHC | CALBIOCHEM 178615 | 1:150 |
| Rat anti-mouse CD31 (Dianova) | IHC | DIA-310-M Clone SZ31 | 1:50-100 |
| Rabbit anti- cow Cytokeratin (Dako) | IHC | Code Z0622 | 1:100 |
| Goat anti-mouse SP-B (Santa Cruz) | IHC | Sc-7704 | 1:100 |
| Rabbit anti-mouse uteroglobin (Abcam) | IHC | Ab40873 | 1:200 |
| Rabbit anti- CFTR (Abcam) | IHC | Ab-59394 | 1:50-100 |
| Rat anti-mouse Sca-1 (Abcam) | IHC | Ab51317 | 1:200 |
| Rabbit anti-mouse Pro-Sp-C (Abcam) | IHC | Ab40879 | 1:200 |
| Anti- mouse Sca-APC-Cy7 (Biolegend) | FACS | 108126 | 1µl/10⁶ cells |
| Anti- mouse Sca-Pacific Blue (Biolegend) | FACS | 108120 | 1µl/10⁶ cells |
| Anti- mouse Sca-APC (Biolegend) | FACS | 108112 | 1µl/10⁶ cells |
| Anti- mouse CD45 APC-Cy7 (Biolegend) | FACS | 103116 clone 30-F11 | 1µl/10⁶ cells |
| Anti- mouse CD45 PE (Biolegend) | FACS | 103106 | 1µl/10⁶ cells |
| Anti- mouse CD31 APC (Biolegend) | FACS | 102510 | 1µl/10⁶ cells |
| Anti- mouse CD31 PE-Cy7 (Biolegend) | FACS | 102418 | 1µl/10⁶ cells |
| Anti- mouse CD31 PE (Biolegend) | FACS | 102408 | 1µl/10⁶ cells |
| Anti- mouse CD326 Percp-Cy5.5 (Ep-CAM) (Biolegend) | FACS | 118220 | 1µl/10⁶ cells |
| Anti- mouse CD326 APC-Cy7 (Ep-CAM) (Biolegend) | FACS | 118218 | 1µl/10⁶ cells |
| Anti- mouse CD24 PE-Cy7 (Biolegend) | FACS | 101822 | 1µl/10⁶ cells |
| Anti- mouse CD24 Pacific Blue (Biolegend) | FACS | 101820 | 1µl/10⁶ cells |
| Anti- mouse CD24 FITC (Biolegend) | FACS | 101806 | 1µl/10⁶ cells |
| Anti- mouse CD49f Pacific Blue (Biolegend) | FACS | 313620 | 1µl/10⁶ cells |
| Anti- mouse CD104 FITC (Biolegend) | FACS | 123606 | 1µl/10⁶ cells |
| Anti- mouse CD90 Pacific Blue (Biolegend) | FACS | 105324 | 1µl/10⁶ cells |
| Anti- mouse CD90 APC (Biolegend) | FACS | 105312 | 1µl/10⁶ cells |
| Anti- mouse CD200 APC (Biolegend) | FACS | 123810 | 1µl/10⁶ cells |
| Anti- mouse CD140a APC (Biolegend) | FACS | 135908 | 1µl/10⁶ cells |
| Anti- mouse CD140a PE (Biolegend) | FACS | 135906 | 1µl/10⁶ cells |
| Anti- mouse Podoplanin APC (Biolegend) | FACS | 127410 | 1µl/10⁶ cells |
| Anti- mouse Podoplanin PE (Biolegend) | FACS | 127408 | 1µl/10⁶ cells |
| Anti- mouse Podoplanin PE-Cy7 (Biolegend | FACS | 127412 | 1µl/10⁶ cells |
| | | | |

| **Secondary antibodies*** | | | |
|---|---|---|---|
| Anti-chicken Alexa Fluor 488 | IHC | 703-545-155 | 1:200 |
| Anti- rabbit Rhodamine Red | IHC | 711-295-152 | 1:200 |
| Anti- rabbit Alexa Fluor 647 | IHC | 711-605-152 | 1:200 |
| Anti- rat Alexa Fluor 594 | IHC | 712-585-150 | 1:200 |
| Anti-goat Alexa Fluor 488 | IHC | 705-545-003 | 1:200 |
| Anti-goat Alexa Fluor 594 | IHC | 705-585-003 | 1:200 |
| Anti-goat AMCA | IHC | 705-155-003 | 1:200 |

| | | | |
|---|---|---|---|
| *All the secondary antibodies were produced in donkey and purchased from Jackson ImmunoResearch or Abcam (unless otherwise indicated). All the information about the antibodies, their specificity, cross-reactivity, application and isotype controls is available in the manufacturers' websites. | | | |

### Removal of CD45⁺ embryonic lung cells by MACS.

Adult lung single cell suspensions were prepared by enzymatic digestion and in some experiments depleted CD45⁺ cells by MACS using LS columns (Miltenyi Biotec) in MACS buffer (0.5 % BSA, 2 mM EDTA in sterile 1 × PBS, filtered and degassed) according to the protocol provided by the vendor. CD45⁺ cells were depleted by treating cells by binding with anti-CD45 magnetic beads (Miltenyi Biotec). Depleted cell populations were analyzed by FACS, plated on GFR Matrigel (BD) for colony-forming assay as indicated in Results.

### In vitro cell colony-forming assay

Epithelial cell colony-forming assay was performed according to a previously published protocol [Bartoncello and McQualter Stem cell biology (2011) 2G.1.1-2G.1.12] with some modifications. Briefly, following initial isolation, lung digestion was performed by finely mincing tissue with a razor blade in the presence of 0.1 % collagenase, and 2.4 U/ml dispase (Roche Diagnostics, Indianapolis, IN) in PBS Ca⁺Mg⁺, followed by incubation at 37 °C for 30 minutes. Nonspecific debris were removed by sequential filtration through 100-µm filters. Whole lung suspensions were washed in 2 % FCS in 1 × PBS. The resulting single cell suspension was resuspended in 100 µl of growth factor-reduced (GFR) Matrigel (BD Biosciences) prediluted 1:1 (vol/vol) with Epi-CFU medium and cultured in a 12 well Transwell plate (1-1.5 × 10⁵ cells per well; Transwell Permeable Supports 0.4 µm, Corning), as described [Bartoncello and McQualter (2011), supra]. The absolute number of epithelial clones was determined after 7-20 days in culture. The growing clones were further characterized as described below. All cell cultures were carried out at 37 °C in a 7 % CO₂ humidified incubator. The medium was replaced every 48-72 hours.

Morphologic and phenotypic characterization of epithelial organoids grown in culture as described above was carried out using whole mount bright field or fluorescence immunohistochemistry. In brief, 3D structures grown under culture conditions were fixed and permeabilized in a 1:1 mixture of methanol and acetone for 20 minutes at 4 °C. Then, the whole mount cultures were washed briefly in 1 × PBS and permeabilized and blocked using 0.5 % Triton X-100 and 10 % horse serum in PBS for 2 hours. Following permeabilization and blocking, the cultures were washed three times with PBS with 0.05 % Tween for 20 minutes, followed by incubation with primary antibodies for 48-72 hours at 4 °C. Following staining with primary antibodies, the whole mount cultures were washed with PBS and 0.5 % Triton-X100 solution, and stained with relevant secondary antibodies overnight at 4 °C, followed by counterstaining with Hoechst dye. Whole mount cultures were assessed by Nikon Eclipse Ti inverted spinning disc microscope. Images were acquired using Andor iQ software, and reconstructed in three dimensions with Imaris software (Bitplane AG, Switzerland, www.bitplane.com).

### Assessment of GFP⁺ foci in chimeric lungs by morphometry

Lungs were fixed with a 4 % PFA solution introduced through the trachea under a constant pressure of 20 cm H₂O. Then the lungs were immersed in fixative overnight at 4 °C. Lungs were processed after PFA treatment and fixed in 30 % sucrose and frozen in Optimal Cutting Temperature (OCT) compound (Sakura Finetek USA, Inc.Tissue-Tek.). Serial step sections, 12 µm in thickness, were taken along the longitudinal axis of the lobe. The fixed distance between the sections was calculated to allow systematic sampling of at least 20 sections across the whole lung. Lung slices were analyzed by fluorescence microscopy. The actual number of GFP⁺ foci (a group of more than 5 distinct GFP⁺ cells was defined as a single patch) was counted per slice using Image Pro software (Media Cybernetics, Crofton, MD, www.mediacy.com). The area of each slice was estimated and calculated by Fiji software (ImageJ, https://fiji.sc). The average size of each GFP⁺ patch was calculated, using random slices from host mice transplanted with 1 x 10⁶ to 3 x 10⁶ adult GFP⁺ lung cells, in which the GFP⁺ patches were distinct from one another (Figures 9A-E). After the average GFP⁺ patch size was determined, the number of GFP⁺ patches was calculated for different cell doses (total high GFP⁺ area / average area of GFP⁺ patch (mm2)), assuming that the frequency per area in a large number of slices reflects distribution per volume.

### Calculation of GFP⁺ engrafted area

Serial slices of chimeric lungs from different time points after transplantation were prepared and stained with anti-GFP antibody in combination with other markers as indicated in the Examples section below. Serial step sections, 12 µm in thickness, were taken along the longitudinal axis of the lobe. The fixed distance between the sections was calculated to allow systematic sampling of at least 20 sections across the whole lung. Slices were obtained using Olympus fluorescent microscope and Olympus digital camera (DP70) with ×10 objective. Each analyzed image was individually evaluated for validation of the staining pattern before processing. As large GFP-positive patches containing hundreds of cells were identified in chimeric lungs, the engrafted area was calculated by Fiji software. The green channel was extracted from the RGB image. The percentage of the engrafted area was then calculated from the whole lung tissue. Engrafted areas had high values of green intensity, whereas the whole lung tissue had low (autofluorescence) green intensity, and air space areas appeared dark. Whole lung tissue excluding air spaces filling the lung structure was detected by applying Gauss blur to the green channel (sigma = 3), setting a low fixed threshold on the blurred image and further smoothing the edges to remove small artifacts using the dilation and erosion operations. A mask of the whole lung tissue (Figures 9A-E) was created and measured its area. The RenyiEntropy global thresholding method [Kapur, et al. Comput. Vis. Graph. Image Process. (1985) 29: 273-285] was used for calculating the intensity threshold of high GFP (engrafted) area and to create a mask (Figures 11A-G and 12A-D). The percentage of engrafted GFP⁺ area was calculated from two measurements as high GFP⁺ area (engrafted) / low GFP⁺ area (total tissue) X 100. In most experiments, automated software was used, but in a few instances, manual examination of the slides was required. However, in all cases, the reader was blinded to the identity of the sample.

### Colocalization analysis

Colocalization analysis was performed on fluorescent sections using Imaris 7.7.2 software (Bitplane AG, Switzerland, www.bitplane.com). Multiple serial slices from chimeric lungs were co-stained with (green) anti-GFP antibody and one of a number of markers used for colocalization analysis (always red). The images for analysis were obtained using an Olympus fluorescent microscope and Olympus digital camera (DP70) with ×40 objective. Each analyzed image was individually evaluated for validation of the pattern of the staining used before processing. The images were opened in the Imaris colocalization module, and the region of interest (ROI) for colocalization was defined by the green channel to concentrate on the relevant parts of the image, defining the whole GFP⁺ area as 100 %. A colocalized channel was created; channel statistics were calculated and exported to Excel files. The extent of colocalization was assessed as the percentage (%) of colocalized ROI material: (total red intensity in colocalized region / total green intensity in the ROI) × 100. The data are presented as mean ± s.d. of all analyzed images for each marker.

### Two-photon microscopy

Mice were euthanized before imaging. Lungs were excised and placed under a glass-covered imaging chamber. Imaging was performed using an Ultima Multiphoton Microscope (Prairie Technologies Middleton, WI) incorporating a pulsed Mai Tai Ti-Sapphire laser (Newport Corp, CA). The laser was tuned to 850-900 nm to either excite EGFP, or to simultaneously excite EGFP and tdTomato. A water-immersed x20 (NA 0.95) or x40 objective (NA 0.8) or x10 air objective (NA 0.3) from Olympus was used. To create a typical Z stack, sections of the lung containing GFP-labeled cells (the donor embryos express GFP under the β-actin promoter; thus all engrafted cells were GFP-positive) were scanned at a depth of approximately 30-150 µm with 1 µm z-steps. The data were analyzed using Imaris software (Bitplane AG, Switzerland, www.bitplane.com). All 3D rendering of the images was performed using Imaris software.

### Assessment of airway hyper-responsiveness (AHR)

Mice were anesthetized using ketamine/xylazine, tracheostomized, and ventilated with a FlexiVent apparatus (SCIREQ, Montreal, Quebec, Canada). After baseline determination of airway resistance, mice were challenged with 0 to 64 mg/mL methacholine nebulized directly into the ventilatory circuit using an AeroNebLab nebulizer (SCIREQ). Two models of respiratory mechanics were used to assess lung resistance (R): the linear first-order single compartment model and the constant-phase model. All data points were collected with FlexiVent software (SCIREQ). Results were expressed as relative increase in R over base line values.

### Statistical analysis

Differences between groups were evaluated by using a t-test. For each data set, mean ± s.d. was calculated and is presented in the Results section of the main text. The differences between the groups were considered statistically significant for P ≤ 0.05.

### EXAMPLE 1

### The fetal lung as a source for hematopoietic stem cells for induction of transplantation tolerance and chimerism

Inventors of the present invention previously shown in a syngeneic mouse model that upon pre-conditioning with naphthalene and total body irradiation (TBI) intravenous infusion of a single cell suspension of canalicular lung cells induced marked long-term lung chimerism [Rosen, C. et al., Nat Med (2015) 21(8): p. 869-79]. Based on this proof of concept, the present inventors have attempted to attain a similar level of lung chimerism following transplantation of canalicular embryonic lung cells from fully mis-matched allogeneic donors. In general, acceptance of such transplants can be attained by using continuous immune suppression. However, considering the problematic side effects associated with chronic immune suppression, an alternative approach such as that based on induction of immune tolerance is more desirable. It is well established that immune tolerance can be induced following transplantation of hematopoietic stem cells which if engrafted can colonize the thymus and lead to central tolerance by negative selection.

The present inventors now found that the canalicular embryonic lung, which comprises not only epithelial lung progenitors but also a marked level of hematopoietic stem cells, can induce durable and multi-lineage hematopoietic chimerism. Specifically, a population of lung hematopoietic progenitors similar to that found in adult bone marrow (BM) and gestational stage (E16) liver can be distinguished by FACS analysis of well-established markers of hematopoietic progenitors (known as LSK cells - Lineage negative, SCA1⁺, c-KIT⁺, as well as the SLAM cells - Lineage negative CD41⁻ CD48⁻ CD150⁺) (Figures 1A-1F). Using a competitive self-renewal assay it was illustrated that the lung hematopoietic progenitors can effectively compete with normal adult bone marrow stem cells (Figures 2A-E).

Accordingly, as illustrated in Figure 3, a new sub-lethal conditioning protocol was established comprising preconditioning the recipient animal with *in vivo* T cell debulking (6 days prior to transplantation), naphthalene (3 days prior to transplantation) and total body irradiation (TBI, 1 day prior to transplantation). The recipient animals were then administered donor-derived single cell suspension of T cell depleted lung cells followed by short-term immunosuppression with cyclophosphamide 3 and 4 days following transplantation. This protocol attained a durable immune tolerance and lung chimerism for the allogeneic embryonic lung cells (Figures 4A-C, 4J and 5A-F) by virtue of HSC that are present in the canalicular lung. Notably, the lung hematopoietic progenitors were capable of inducing chimerism in the recipient peripheral blood (Figures 4A-G) as well as in the recipient's bone marrow (Figures 4H-L). Taken together, the lung hematopoietic progenitors are capable of inducing central tolerance to the transplanted lung cells which enable a long-lasting engraftment in the absence of chronic immunosuppression.

### EXAMPLE 2

### Induction of hematopoietic chimerism after transplantation of fresh adult lung cells

Adult lung cells were harvested from a GFP positive C57BL/6 mouse and a single cell suspension comprising 8 x 10⁶ was injected i.v. into C57BL/6 recipient mice following conditioning with NA and 6 GY TBI.

Eight weeks after transplantation lung tissue from transplanted mice was obtained, fixed and analyzed for GFP positive patches. As evident from the results (Figures 6A-F) marked number of donor derived GFP positive patches were found in the recipient's lungs. Furthermore, blood analysis revealed induction of blood chimerism by infusion of adult lung cells similar to that obtained by E16 fetal lung cells (Figures 7A-J).

### EXAMPLE 3

### Induction of lung chimerism after transplantation of ex vivo expanded lung cells

Lung cells were expanded *ex vivo* by first obtaining GFP positive C57BL E16 lung cells and seeding the cells on tissue culture plates with a suitable condition medium (iMEF) together with epithelial growth factor and Rock inhibitor. A single cell suspension comprising 2 x 10⁶ expanded cells were injected i.v. into C57BL/6 recipient mice following conditioning with NA and 6 GY TBI.

Eight weeks after transplantation lung tissue from transplanted mice was obtained, fixed and analyzed for GFP positive patches. As evident from the results (Figures 8A-F) marked number of donor derived GFP positive patches were found in the recipient's lungs.

### EXAMPLE 4

### FACS characterization of progenitors in the adult mouse lung

The present inventors characterized the cellular composition of fetal versus adult mouse lung. As can be expected, the cell composition of the adult mouse lung is different from E16 fetal lung cells (Figure 16A). The adult lung exhibited higher levels of CD31⁺ endothelial and CD45⁺ hematopoietic cells, as well as of CD45⁻ CD31⁻ Ep⁻ Cam⁻ SCA-1⁺ PDGR⁻ alpha⁺ mesenchymal progenitors (1.9 ± 0.29 % vs 0.1 ± 0.05 %) (Figures 16B, 16D), while the concentration of CD45⁻ CD31⁻ Ep⁻ Cam⁺ CD24⁺ mouse epithelial progenitors (Figures 16 B-C) was lower in the adult lung (1.13 ± 0.05% vs. 8.1 ± 1).

### EXAMPLE 5

### In vitro differentiation of adult lung cells

To evaluate the potential regenerative activity of the epithelial lung progenitors within the adult lung preparation, the present inventors initially tested their ability to form lung organoids *ex-vivo* (Figure 17A). After removal of CD45⁺ hematopoietic cells, the remaining adult lung cells were plated on chamber plates covered with Matrigel, and 2-3 weeks later, the cultures were observed for the presence of lung organoids. The plates containing the organoids were fixed and stained for different lung differentiation markers. Although a smaller number of organoids was obtained compared to fetal lung cells, both alveolar and bronchiolar organoids could be clearly detected (Figures 17B-C).

Furthermore, not only epithelial cells (CK⁺ or ATI⁺) could be found within the growing organoids, but Nestin⁺ and Sca1⁺ mesenchymal cells were observed as well (Figures 17D-G).

### EXAMPLE 6

### Patch forming capacity of adult lung cells

The preliminary results described above strongly suggested that adult lung progenitors might offer an additional source for transplantation. However, considering the limitations of *ex-vivo* assays, it was critical to verify this possibility by using present newly developed assay for 'patch' forming progenitors *in vivo.*

As previously described by Rosen et al. [Rosen, C. et al., Nat Med (2015) supra], this assay, based on transplantation of GFP⁺ E16 fetal lung progenitors into recipients conditioned with naphthalene and 6 GY TBI, leads to formation of discrete clonogenic patches likely derived from a single progenitor. Considering that fetal lungs exhibit higher levels of CD45⁻ CD31⁻EPCAM⁺CD24⁺ epithelial progenitors, the present inventors initially chose to use a 4-fold greater cell number for adult lung transplantation. Thus the present inventors used 4 x 10⁶ adult lung cells from C57BL-GFP donors, and evaluated chimerism in the recipients' lungs 8 weeks after transplantation. As shown in Figure 9A, histological staining revealed GFP⁺ patches in the host lung, indicating that adult lung indeed contains patch forming progenitors.

In order to compare quantitatively the frequency of these progenitors in adult versus fetal lungs, the present inventors conducted a dose response experiment, in which 1 to 8 x 10⁶ adult GFP positive cells from C57BL donor mice were transplanted after conditioning of the recipients with naphthalene, and 6 GY TBI 48 hours later. As a positive control, 1 x 10⁶ GFP fetal lung cells were administered. Mice were sacrificed 8 weeks after transplantation, and their lungs were evaluated by immunohistology. Using the FIJI software, capable of distinguishing between different fluorescence intensities, the level of GFP intensity was recorded and analyzed throughout the lung (Figures 9B-E). Black area in the host lung indicates the background of the scanned field representing an empty area without cells. Weak green indicates GFP negative host cells (auto-fluorescence), while intense green staining marks donor derived GFP positive cells. The software calculation ignores the black area of the background and can estimate the percentage of the GFP high level area out of the total cellular region.

One caveat regarding this calculation is related to patch size, which increases proportionally upon cell dose escalation. Thus, at high cell doses, it becomes increasingly difficult to define the patch borders and the observed patches might include two or three neighboring patches. To address this problem, the present inventors attempted to define the average size of a single GFP patch observed at low cell dose at which single patches can be clearly delineated, and this average area was used to calculate the number of patches formed following transplantation at high cell dose. Thus, the number of patches in a total area of 2 x 2 x 1mm³ is calculated for each lung. Again, as can be seen in Figures 10A-B, this form of analysis showed linear dependence on cell dose.

Taken together, this analysis suggested that transplantation of about 3 x 10⁶ adult donor lung cells results quantitatively in the same level of chimerism found following transplantation of 1 x 10⁶ E16 fetal lung cells, indicating that the frequency of patch forming cells is about 3 fold lower that that found in the E16 fetal lung.

### EXAMPLE 7

### Three dimensional analysis by two photon microscopy of donor-derived patches

To visualize the integration of GFP⁺ donor derived cells into the 3-dimensional structure of the lung, a two-photon microscopy was used, immediately after sacrifice of the transplanted mice (SI 3).

Notably, as previously shown for E16 lung cells, only discrete green or red without any yellow patches were found after transplantation of a 1:1 mixture of green GFP and red TdTomato positive adult lung cells (Figures 11A-G), strongly indicating that each patch is derived from a single lung progenitor.

### EXAMPLE 8

### Lung localization of donor-derived patches

In general, donor derived patches can be classified by their anatomical location within the lung, namely, bronchiolar, bronchioalveolar and alveolar patches. As can be seen in Figures 12A-D, transplantation of adult lung cells leads predominantly to alveolar patches similarly to the results with fetal lung cells. However, bronchiolar and bronchioalveolar patches were also detected, although there was a trend for a more pronounced level of such patches following fetal lung transplantation.

### EXAMPLE 9

### Immunohistological analysis of donor-derived lung patches

The present inventors next characterized the cell types formed within the adult lung-derived patches. As can be seen in Figures 13A-J, immunohistological analysis revealed the presence of epithelial (CK⁺) (Figures 13A-C and 13J), endothelial (CD31⁺) (Figures 13D-F and 13J), and mesenchymal (Nestin⁺) (Figures 13G-J) cells within each patch.

Within the epithelial lineage, this analysis revealed both types of differentiated alveolar cells, namely, alveolar type I cells (ATI), responsible for gas exchange between the alveoli and the blood, and alveolar type II (ATII) cells responsible for surfactant secretion. Antibody against aquaporin 5, a major water channel expressed in ATI cells, was used to identify ATI cells (Figures 14A-C) while surfactant protein C was used to stain ATII cells (Figures 14D-F). Another important cell type is the Club cell, found in the bronchioles; these cells protect the bronchiolar epithelium, by secreting proteins such as uteroglobin and other components similar to surfactant. Club cells, identified by staining for the CCSP protein (CC16, Figures 14G-I) are also responsible for detoxifying harmful substances inhaled into the lungs. Finally, as shown in Figures 14J-L, the present inventors also found colocalization of the cystic fibrosis transmembrane conductance regulator (CFTR) within the GFP⁺ donor derived cells, indicating that, as in E16 cell transplants, the transplanted cells can potentially provide functions missing in diseased lung of cystic fibrosis patients.

### EXAMPLE 10

### Functional assessment of lung injury repair

The marked lung colonization with donor-derived cells within different lung cell lineages (Figures 18A-I and 19A-L) strongly indicated that the current transplantation procedure could offer a new modality for lung regeneration. To test the functional capacity of the transplanted cells, the Dynamic Resistance (R) of the lung was measured following methacholine challenge (64 mg/ml). As can be seen in Figure 15, this parameter, which quantitatively assesses the level of constriction in the lungs, is significantly reduced after exposure to naphthalene plus 6 GY TBI (P=0.0004) and was completely restored 8 weeks after transplantation of 4 x 10⁶ adult lung cells (P=0.0275).

## Claims

1. Non-syngeneic pulmonary tissue cells in suspension comprising an effective amount of hematopoietic precursor cells (HPCs) or supplemented with HPCs, wherein said effective amount is a sufficient amount to achieve tolerance to said pulmonary tissue cells in the absence of chronic immunosuppressive regimen, and wherein said effective amount of said HPCs comprises at least about 1 x 10⁵ cells per Kg body weight of a subject, for use in treating a pulmonary disorder or injury in a subject in need thereof or for inducing donor specific tolerance in a subject in need of a pulmonary cell or tissue transplantation.

2. The non-syngeneic pulmonary tissue cells in suspension for use of claim 1, further comprising a sublethal, lethal or supralethal conditioning protocol.

3. The non-syngeneic pulmonary tissue cells in suspension for use of claim 2, wherein said conditioning protocol comprises a reduced intensity conditioning (RIC) and optionally wherein said conditioning protocol comprises at least one of *in-vivo* T cell debulking, total body irradiation (TBI), total lymphoid irradiation (TLI), partial body irradiation, a chemotherapeutic agent and/or an antibody immunotherapy.

4. The non-syngeneic pulmonary tissue cells in suspension for use of any one of claims 1-3, further comprising an agent capable of inducing damage to the pulmonary tissue, wherein said damage results in proliferation of resident stem cells in said pulmonary tissue.

5. The non-syngeneic pulmonary tissue cells in suspension for use of claim 4, wherein said agent capable of inducing damage to the pulmonary tissue comprises naphthalene or cyclophosphamide.

6. The non-syngeneic pulmonary tissue cells in suspension for use of any one of claims 1-5, further comprising the use of an immunosuppressive agent for up to two weeks following said non-syngeneic pulmonary tissue cells in suspension, and optionally wherein said immunosuppressive agent comprises cyclophosphamide, and optionally wherein said cyclophosphamide:
(i) is for a single dose administration; or
(ii) is for a two dose administration.

7. The non-syngeneic pulmonary tissue cells in suspension for use of claim 6, wherein each of said two doses:
(i) comprises a concentration of about 50-150 mg per kg body weight; and/or
(ii) is effected on days 3 and 4 following said non-syngeneic pulmonary tissue cells in suspension.

8. The non-syngeneic pulmonary tissue cells in suspension for use of any one of claims 1-7, wherein said pulmonary tissue cells comprise:
(i) mammalian pulmonary cells, and optionally wherein said mammalian pulmonary cells are human cells; and/or
(ii) fetal pulmonary cells, and optionally wherein said fetal pulmonary cells are from a fetus at a developmental stage corresponding to that of a human pulmonary organ/tissue at a gestational stage selected from a range of about 20 to about 22 weeks of gestation; and/or
(iii) adult pulmonary cells; and/or
(iv) de-differentiated cells; and/or
(v) *ex vivo* expanded cells.

9. The non-syngeneic pulmonary tissue cells in suspension for use of any one of claims 1-8, wherein said pulmonary tissue cells in suspension are:
(i) at a dose of at least about 10 x 10⁶ cells per kilogram body weight of the subject; or
(ii) at a dose of at least about 40 x 10⁶ cells per kilogram body weight of the subject; or
(iii) at a dose of at least about 100 x 10⁶ cells per kilogram body weight of the subject; or
(iv) are depleted of T cells.

10. The non-syngeneic pulmonary tissue cells in suspension for use of any one of claims 1-9, wherein said HPCs comprise:
(i) CD34⁺ cells and/or signaling lymphocytic activation molecule (SLAM) cells; and/or
(ii) at least about 1 x 10⁶ cells per kilogram body weight of the subject; and/or
(iii) at least about 10 x 10⁶ cells per kilogram body weight of the subject.

11. The non-syngeneic pulmonary tissue cells in suspension for use of any one of claims 1-10, wherein said supplemented with said HPCs comprises:
(i) the addition of HPCs obtained from the same donor as said pulmonary tissue; and/or
(ii) said pulmonary tissue cells in the same formulation as said HPCs; or
(iii) said pulmonary tissue cells in a separate formulation than said HPCs.

12. The non-syngeneic pulmonary tissue cells in suspension for use of any one of claims 1-11, wherein said pulmonary tissue cells in suspension comprise a heterogeneous population of cells.

13. The non-syngeneic pulmonary tissue cells in suspension for use of any one of claims 1-12, formulated for an intravenous route or an intratracheal route of administration.

14. The non-syngeneic pulmonary tissue cells in suspension for use of any one of claims 1-13, wherein said subject in need of said pulmonary cell or tissue transplantation has a pulmonary disorder or injury, and optionally wherein said pulmonary disorder or injury comprises chronic inflammation of the lungs.

## Patentansprüche

1. Nicht-syngene Lungengewebezellen in Suspension, umfassend eine wirksame Menge an hämatopoetischen Vorläuferzellen (HPCs) oder ergänzt mit HPCs, wobei die genannte wirksame Menge eine ausreichende Menge ist, um eine Toleranz gegenüber den genannten Lungengewebezellen in Abwesenheit eines chronischen immunsuppressiven Regimes zu erreichen, und wobei die genannte wirksame Menge der genannten HPCs mindestens etwa 1 x 10⁵ Zellen pro kg Körpergewicht eines Probanden zur Verwendung bei der Behandlung einer Lungenerkrankung oder -verletzung bei einem Probanden, der dies benötigt, oder zum Induzieren einer spenderspezifischen Toleranz bei einem Probanden umfasst, der eine Lungenzell- oder Gewebetransplantation benötigt.

2. Nicht-syngene Lungengewebezellen in Suspension zur Verwendung nach Anspruch 1, ferner umfassend ein subletales, letales oder supraletales Konditionierungsprotokoll.

3. Nicht-syngene Lungengewebezellen in Suspension zur Verwendung nach Anspruch 2, wobei das genannte Konditionierungsprotokoll eine Konditionierung mit reduzierter Intensität (RIC) umfasst, und gegebenenfalls wobei das genannte Konditionierungsprotokoll mindestens eines von in vivo-T-Zell-Debulking, Ganzkörperbestrahlung (TBI), Gesamtlymphoidbestrahlung (TLI), Teilkörperbestrahlung, einem Chemotherapeutikum und/oder einer Antikörperimmuntherapie umfasst.

4. Nicht-syngene Lungengewebezellen in Suspension zur Verwendung nach einem der Ansprüche 1-3, ferner umfassend ein Mittel, das zum Induzieren einer Schädigung des Lungengewebes in der Lage ist, wobei die genannte Schädigung zur Proliferation residenter Stammzellen in dem genannten Lungengewebe führt.

5. Nicht-syngene Lungengewebezellen in Suspension zur Verwendung nach Anspruch 4, wobei das genannte Mittel, das zum Induzieren einer Schädigung des Lungengewebes in der Lage ist, Naphthalin oder Cyclophosphamid umfasst.

6. Nicht-syngene Lungengewebezellen in Suspension zur Verwendung nach einem der Ansprüche 1-5, ferner umfassend die Verwendung eines Immunsuppressivums für bis zu zwei Wochen nach den genannten nicht-syngenen Lungengewebezellen in Suspension und gegebenenfalls wobei das genannte Immunsuppressivum Cyclophosphamid umfasst, und gegebenenfalls wobei das genannte Cyclophosphamid:
(i) für eine Verabreichung von einer Dosis ist; oder
(ii) für eine Verabreichung von zwei Dosen ist.

7. Nicht-syngene Lungengewebezellen in Suspension zur Verwendung nach Anspruch 6, wobei jede der genannten zwei Dosen:
(i) eine Konzentration von etwa 50-150 mg pro kg Körpergewicht umfasst; und/oder
(ii) an den Tagen 3 und 4 nach den genannten nicht-syngenen Lungengewebezellen in Suspension erfolgt.

8. Nicht-syngene Lungengewebezellen in Suspension zur Verwendung nach einem der Ansprüche 1-7, wobei die genannten Lungengewebezellen umfassen:
(i) Lungenzellen von Säugern und gegebenenfalls wobei die genannten Lungenzellen von Säugern menschliche Zellen sind; und/oder
(ii) fetale Lungenzellen und gegebenenfalls wobei die genannten fetalen Lungenzellen von einem Fötus in einem Entwicklungsstadium stammen, das dem eines menschlichen Lungenorgans/-gewebes in einer Schwangerschaftsphase entspricht, ausgewählt aus einem Bereich von etwa 20 bis etwa 22 Schwangerschaftswochen; und/oder
(iii) adulte Lungenzellen; und/oder
(iv) dedifferenzierte Zellen; und/oder
(v) ex *vivo*-expandierte Zellen.

9. Nicht-syngene Lungengewebezellen in Suspension zur Verwendung nach einem der Ansprüche 1-8, wobei die genannten Lungengewebezellen:
(i) bei einer Dosis von mindestens etwa 10 x 10⁶ Zellen pro Kilogramm Körpergewicht des Probanden; oder
(ii) bei einer Dosis von mindestens etwa 40 x 10⁶ Zellen pro Kilogramm Körpergewicht des Probanden; oder
(iii) bei einer Dosis von mindestens etwa 100 x 10⁶ Zellen pro Kilogramm Körpergewicht des Probanden; oder
(iv) an T-Zellen verarmt sind.

10. Nicht-syngene Lungengewebezellen in Suspension zur Verwendung nach einem der Ansprüche 1-9, wobei die genannten HPCs umfassen:
(i) CD34⁺-Zellen und/oder Signalzellen für lymphozytische Aktivierungsmoleküle (SLAM); und/oder
(ii) mindestens etwa 1 x 10⁶ Zellen pro Kilogramm Körpergewicht des Probanden; und/oder
(iii) mindestens etwa 10 x 10⁶ Zellen pro Kilogramm Körpergewicht des Probanden.

11. Nicht-syngene Lungengewebezellen in Suspension zur Verwendung nach einem der Ansprüche 1-10, wobei die genannten, mit den genannten HPCs ergänzten umfasst:
(i) das Zugeben von HPCs, die von demselben Spender wie das genannte Lungengewebe erhalten wurden; und/oder
(ii) die genannten Lungengewebezellen in der gleichen Formulierung wie die genannten HPCs; oder
(iii) die genannten Lungengewebezellen in einer anderen Formulierung als die genannten HPCs.

12. Nicht-syngene Lungengewebezellen in Suspension zur Verwendung nach einem der Ansprüche 1-11, wobei die genannten Lungengewebezellen in Suspension eine heterogene Population von Zellen umfassen.

13. Nicht-syngene Lungengewebezellen in Suspension zur Verwendung nach einem der Ansprüche 1-12, formuliert für einen intravenösen Weg oder einen intratrachealen Verabreichungsweg.

14. Nicht-syngene Lungengewebezellen in Suspension zur Verwendung nach einem der Ansprüche 1-13, wobei der genannte Proband, der die genannte Lungenzell- oder Gewebetransplantation benötigt, eine Lungenstörung oder -verletzung aufweist, und gegebenenfalls wobei die genannte Lungenstörung oder -verletzung eine chronische Entzündung der Lunge umfasst.

## Revendications

1. Cellules de tissu pulmonaire non syngéniques en suspension comprenant une quantité efficace de cellules précurseurs hématopoïétiques (HPC) ou complétées par des HPC, ladite quantité efficace étant une quantité suffisante pour atteindre la tolérance auxdites cellules de tissu pulmonaire en l'absence de régime immunosuppresseur chronique et ladite quantité efficace desdites HPC comprenant au moins environ 1 x 10⁵ cellules par kg de poids corporel d'un sujet, pour une utilisation dans le traitement d'un trouble pulmonaire ou d'une blessure chez un sujet qui en a besoin ou pour induire une tolérance spécifique du donneur chez un sujet ayant besoin d'une transplantation de cellules ou de tissus pulmonaires.

2. Cellules de tissu pulmonaire non syngéniques en suspension utilisables selon la revendication 1, comprenant en outre un protocole de conditionnement sublétal, létal ou supralétal.

3. Cellules de tissu pulmonaire non syngéniques en suspension utilisables selon la revendication 2, dont ledit protocole de conditionnement comprend un conditionnement à intensité réduite (RIC) et, éventuellement, dont ledit protocole de conditionnement comprend le *démoulage in vivo des* lymphocytes T, l'irradiation totale du corps (TBI), une irradiation lymphoïde totale (TLI), une irradiation corporelle partielle, un agent chimiothérapeutique et/ou une immunothérapie par anticorps.

4. Cellules de tissu pulmonaire non syngéniques en suspension utilisables selon l'une quelconque des revendications 1 à 3, comprenant en outre un agent susceptible d'induire un dommage au tissu pulmonaire, ledit dommage entraînant la prolifération de cellules souches résidentes dans ledit tissu pulmonaire.

5. Cellules de tissu pulmonaire non syngéniques en suspension utilisables selon la revendication 4, dans lesquelles ledit agent susceptible d'induire des dommages au tissu pulmonaire comprend du naphtalène ou du cyclophosphamide.

6. Cellules de tissu pulmonaire non syngéniques en suspension utilisables selon l'une quelconque des revendications 1 à 5, comprenant en outre l'utilisation d'un agent immunosuppresseur pendant deux semaines maximum après lesdites cellules de tissu pulmonaire non syngéniques en suspension et éventuellement dans lesquelles ledit agent immunosuppresseur comprend du cyclophosphamide et éventuellement dans lesquelles ledit cyclophosphamide :
(i) est destiné à une administration à dose unique ; ou
(ii) est destiné à une administration en deux doses.

7. Cellules de tissu pulmonaire non syngéniques en suspension utilisables selon la revendication 6, dans lesquelles chacune desdites deux doses :
(i) comprend une concentration d'environ 50 à 150 mg par kg de poids corporel ; et/ou
(ii) est effectuée aux jours 3 et 4 après la mise en suspension desdites cellules de tissu pulmonaire non syngéniques.

8. Cellules de tissu pulmonaire non syngéniques en suspension utilisables selon l'une quelconque des revendications 1 à 7, lesdites cellules de tissu pulmonaire comprenant :
(i) des cellules pulmonaires de mammifère, lesdites cellules pulmonaires de mammifère étant éventuellement des cellules humaines ; et/ou
(ii) des cellules pulmonaires fœtales, lesdites cellules pulmonaires fœtales provenant éventuellement d'un fœtus à un stade de développement correspondant à celui d'un organe/tissu pulmonaire humain à un stade gestationnel choisi dans une plage d'environ 20 à environ 22 semaines de gestation ; et/ou
(iii) des cellules pulmonaires adultes ; et/ou
(iv) des cellules dé-différenciées ; et/ou
(v) des cellules expansées *ex vivo.*

9. Cellules de tissu pulmonaire non syngéniques en suspension utilisables selon l'une quelconque des revendications 1 à 8, lesdites cellules de tissu pulmonaire en suspension sont :
(i) à une dose d'au moins environ 10 x 10⁶ cellules par kilogramme de poids corporel du sujet ; ou
(ii) à une dose d'au moins environ 40 x 10⁶ cellules par kilogramme de poids corporel du sujet ; ou
(iii) à une dose d'au moins environ 100 x 10⁶ cellules par kilogramme de poids corporel du sujet ; ou
(iv) sont épuisés en lymphocytes T.

10. Cellules de tissu pulmonaire non syngéniques en suspension utilisables selon l'une quelconque des revendications 1 à 9, dans lesquelles lesdites HPC comprennent :
(i) des cellules CD34⁺ et/ou des cellules de molécule d'activation lymphocytaire de signalisation (SLAM) ; et/ou
(ii) au moins environ 1 x 10⁶ cellules par kilogramme de poids corporel du sujet ; et/ou
(iii) au moins environ 10 x 10⁶ cellules par kilogramme de poids corporel du sujet.

11. Cellules de tissu pulmonaire non syngéniques en suspension utilisables selon l'une quelconque des revendications 1 à 10, dans lesquelles ledit complément avec lesdites HPC comprend :
(i) l'ajout de HPC obtenues du même donneur que ledit tissu pulmonaire ; et/ou
(ii) lesdites cellules de tissu pulmonaire dans la même formulation que lesdites HPC ; ou
(iii) lesdites cellules de tissu pulmonaire dans une formulation distincte de celle desdites HPC.

12. Cellules de tissu pulmonaire non syngéniques en suspension utilisables selon l'une quelconque des revendications 1 à 11, dans lesquelles lesdites cellules de tissu pulmonaire en suspension comprennent une population hétérogène de cellules.

13. Cellules de tissu pulmonaire non syngéniques en suspension utilisables selon l'une quelconque des revendications 1 à 12, formulées pour une voie d'administration intraveineuse ou intratrachéale.

14. Cellules de tissu pulmonaire non syngéniques en suspension utilisables selon l'une quelconque des revendications 1 à 13, ledit sujet nécessitant ladite transplantation de cellules ou de tissu pulmonaires souffrant d'un trouble ou d'une blessure pulmonaire et ledit trouble ou ladite blessure pulmonaire comprenant éventuellement une inflammation chronique des poumons.
